(19) Europäisches Patentamt
European Patent Office
Office européen des brevets

(11) Publication number: 0 369 810 A2

(12) # EUROPEAN PATENT APPLICATION

(21) Application number: 89311925.5

(22) Date of filing: 17.11.89

(51) Int. Cl.5: A61K 31/44, A61K 31/495, A61K 31/34, A61K 31/425, A61K 31/47, A61K 31/55

The title of the invention has been amended (Guidelines for Examination in the EPO, A-III, 7.3).

(30) Priority: 18.11.88 JP 293103/88

(43) Date of publication of application: 23.05.90 Bulletin 90/21

(84) Designated Contracting States: AT BE CH DE FR GB IT LI LU NL SE

(71) Applicant: TAKEDA CHEMICAL INDUSTRIES, LTD.
3-6, Doshomachi 2-chome Chuo-ku
Osaka 541(JP)

(72) Inventor: Tsushima, Susumu
1695 Second Street No. 503
Highland Park, IL 60035(US)
Inventor: Kondo, Koichi
6-3, Kabutodai 3-chome Kizo-cho
Sohraku-gun Kyoto 619-02(JP)
Inventor: Nishikawa, Kohei
5-19, Oharano-Kamisatotorimicho
Nishikyo-ku Kyoto 610-11(JP)

(74) Representative: Laredo, Jack Joseph et al
Elkington and Fife Beacon House 113 Kingsway
London, WC2B 6PP(GB)

(54) **PAF antagonists as anti-hyperendothelinemia agents.**

(57) Platelet activating factor antagonists inhibit the activities of endothelin and are useful as drugs for prevention or treatment of various endothelin-related diseases, hyperendothelinemia.

EP 0 369 810 A2

## ANTI-HYPERENDOTHELINEMIA AGENT

This invention relates to an agent for prevention or treatment of hyperendothelinemia. More particularly, this invention relates to an agent for prevention or treatment of hyperendothelinemia, which contains a platelet activating factor (PAF) antagonist.

### Prior art

Endothelin is a vasoconstrictor peptide which has been recently found and isolated from endothelial cells, and composed of 21 amino acid residues which contain two pairs of S-S bonds [M. Yanagisawa et al., Nature, 332, 411 (1988)]. Endothelin induces a potent vasoconstriction in main blood vessels (coronary artery, basilar artery, mesenteric artery and aorta) in various animals including human. The activities of endothelin are not less than 10 times as potent as those of known vasoconstriction peptides such as angiotensin II, vasopressin and neuropeptide Y, and durable. Receptor antagonists and synthetic antagonists to known vasoactive substances (serotonin, norepinephrine, thromboxane $A_2$, leukotriene) do not produce any effect on the vasoconstriction activities of endothelin, while it is known that $Ca^{2+}$ antagonists inhibit the activities. Further, it is reported that endothelin induces bronchial constriction [Y. Uchida et al., Eur. J. Pharmacol., 154, 227 (1988)].

Furthermore, endothelin causes renal vasoconstriction and reduces renal blood flow, glomerular filtration rate, urine volume and urinary sodium excretion [W. L. Miller et al., J. Clin. Invest., 83, 317 (1989); K. F. Badr et al., J. Clin. Invest., 83, 336 (1989)].

Surprisingly, the present inventors have found that PAF antagonists inhibit the activities of endothelin, and have completed the present invention. Thus, it is known that endothelin elevates the concentration of cytosolic $Ca^{2+}$, and PAF antagonists remarkably inhibit the elevation of cytosolic $Ca^{2+}$. No drug having such effects is known other than the above-mentioned $Ca^{2+}$ antagonists. Further, the present inventors conducted research on the toxicity in view that endothelin has a similar structure to scorpion venom. As the result, it has been found that administration of a large amount of endothelin induces death of the animals, and that the lethality can be protected by PAF antagonists.

As mentioned above, it was not known that PAF antagonists would inhibit the activities of endothelin.

### Disclosure of the invention

The present invention provides an agent for prevention or treatment of hyperendothelinemia, which contains a PAF antagonist.

Any kind of PAF antagonist can be used for the present invention as long as it can inhibit the activity of PAF. As the PAF antagonist, there are mentioned compounds which are disclosed in the following references, for instance:

(i) P. Braquet, L. Touqui, T.Y. Shen and B.B. Vargoftig, Pharmacological Reviews, 39, 97 (1987).

(ii)Ichiro Kudo and Keizo Inoue, Inflammation (Ensho), 7, 309 (1987).

(iii) Hiroshi Honma, Experimental Medicine (Jikken Igaku), 4, 55 (1986).

More specifically, there are mentioned the following compounds.

1) Compounds disclosed in Japanese Unexamined Patent Publication No. 243047/1985.

Lipid derivatives of the formula:

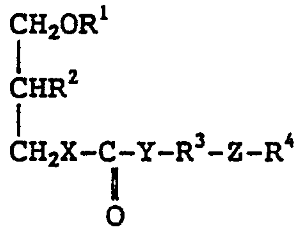

[I]

[wherein $R^1$ is alkyl or alkylcarbamoyl; $R^2$ is hydrogen, hydroxy which may be substituted, amino which may be substituted or cyclic amino; $R^3$ is a chemical binding or alkylene which may be substituted; $R^4$ is hydrogen, alkyl or aralkyl; X and Y are independently O (oxygen atom), S (sulfur atom) or an imino group which may be substituted, and when Y is an imino group, Y, taken together with the imino group represented by X or $R^4$, may form a ring; Z is imino or a nitrogen-containing heterocyclic ring which may be substituted] and salts thereof.

With reference to the above formula [I], the alkyl group represented by $R^1$ may be straight-chain or branched-chain,. and includes alkyl groups having about 10 to 30 carbon atoms, such as decyl, undecyl; tridecyl, tetradecyl, pentadecyl, hexadecyl, heptadecyl, octadecyl, nonadecyl, eicosanyl, heneicosanyl, docosanyl, tricosanyl, tetracosanyl, pentacosanyl, hexacosanyl, heptacosanyl, octacosanyl, nonacosanyl, triacocntanyl, farnesyl and dihydrophytyl; among others, alkyl groups of 14 to 20 carbon atoms are preferable, with alkyl groups of 14 to 18 carbon atoms being more preferable, with alkyl groups of 15 to 18 carbon atoms being still more preferable, with alkyl groups of 16 to 18 carbon atoms being most preferable. In cases in which $R^1$ is an alkylcarbamoyl group, $R^1$ can be expressed as the formula:

$R^5NHCO-$ (i)

wherein $R^5$ includes alkyl groups having about 10 to 30 carbon atoms as is the case with the alkyl groups represented by $R^1$ as mentioned above; among others, preferred are those having an alkyl group having 14 to 20 carbon atoms as the alkyl group in the alkylcarbamoyl group, and those having an alkyl group of 14 to 18 carbon atoms are more preferred, and those having an alkyl group of 15 to 18 carbon atoms are still more preferred and those having an alkyl group of 16 to 18 carbon atoms are most preferred.

As the optionally substituted hydroxyl group represented by $R^2$, there may be mentioned, for example, hydroxy, alkoxy, aralkyloxy, acyloxy or groups of the formula:

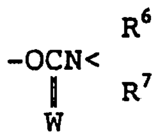

(ii)

[wherein W is an oxygen or sulfur atom; $R^6$ and $R^7$ are independently hydrogen or alkyl, or both, taken together with the adjacent nitrogen atom, form a ring].

As the alkoxy group represented by $R^2$, there may be mentioned lower alkoxy groups having about 1 to 5 carbon atoms, such as methoxy, ethoxy, propoxy, isopropoxy, butoxy, isobutoxy and pentoxy.

As the aralkyloxy group represented by $R^2$, there may be mentioned phenyl-lower($C_{1-5}$)alkoxy, such as benzyloxy, phenethyloxy, $\alpha$-methylbenzyloxy, $\alpha$-methylphenethyloxy and $\beta$-methylphenethyloxy.

As the acyloxy group represented by $R^2$, there may be mentioned lower alkanoyloxy having about 1 to 5 carbon atoms, such as formyloxy, acetyloxy, propionyloxy, butyryloxy, isobutyryloxy, valeryloxy and isovaleryloxy, and acyloxy groups, such as benzoyloxy, phenoxycarbonyloxy and lower-($C_{1-5}$)-alkoxycarbonyloxy (e.g., methoxycarbonyloxy, ethoxycarbonyloxy, propoxycarbonyloxy, butoxycarbonyloxy, etc.).

Referring to the formula (ii), the alkyl group represented by $R^6$ and $R^7$ includes lower alkyl groups having about 1 to 5 carbon atoms, such as methyl, ethyl, propyl, isopropyl, butyl, isobutyl and pentyl, The ring which $R^6$ and $R^7$, taken together with the adjacent nitrogen atom, forms includes 3- to 7-membered heterocyclic rings which may have hetero atoms, such as nitrogen, oxygen and sulfur atoms, in addition to the said nitrogen atom, such as 1-aziridinyl, 1-azetidinyl, 1-pyrrolidinyl, piperidino, 1-perhydroazepinyl, 1-piperazinyl, morpholino, thiomorpholino, 1-perhydrodiazepinyl, 4-perhydrooxazepinyl and 4-perhydrothiazepinyl.

The optionally substituted amino represented by $R^2$ includes, for example, amino and acylamino.

As the acylamino group represented by $R^2$, there may be mentioned lower alkanoylamino having about

1 to 5 carbon atoms, such as formamido, acetamido, propionamido, butanamido, isobutanamido, valeramido and isovaleramido, and acylamino groups, such as benzoylamino.

As the cyclic amino represented by $R^2$, there may be mentioned 3- to 7-membered hetero-mono-cyclic rings, such as 1-aziridinyl, 1-azetidinyl, 1-pyrrolidinyl, piperidino, 1-perhydroazepinyl, 1-piperazinyl, morpholino, thiomorpholino, 1-perhydrodiazepinyl, 4-perhydrooxazepinyl and 4-perhydrothiazepinyl, and condensed rings having about 8 to 9 carbon atoms, such as 2-isoindolinyl. The said heterocyclic and condensed rings may substituted by, for example, one or two oxo groups, at their positions susceptible to substitution, and the substituted hetero-mono-cyclic and condensed rings include, for example, 2,5-dioxopyrrolidinyl and 1,3-dioxoisoindolinyl.

The compounds having alkoxy as $R^2$ are more preferable.

The alkylene chain represented by $R^3$ includes straight-chain or branched-chain alkylene chains having about 1 to 8 carbon atoms, and there may be mentioned, for example, methylene, ethylene, trimethylene, tetramethylene, pentamethylene, hexamethylene, heptamethylene, and octamethylene. The said alkylene chains may be substituted by, for example, lower($C_{1-4}$)alkoxycarbonyl [e.g. methoxycarbonyl, ethoxycarbonyl] or carboxylato, and the case that said substituent is bound to the position adjacent to the carbon atom of the group $R^3$ being bound to the group Z is preferable. As $R^3$, among others, methylene, ethylene and trimethylene are preferable, and methylene or ethylene is more preferable.

The alkyl group represented by $R^4$ includes lower alkyl groups having about 1 to 6 carbon atoms, such as methyl, ethyl, propyl, isopropyl, butyl, isobutyl, sec-butyl, tert-butyl, pentyl and hexyl, and said lower alkyl group may have an unsaturated bond. The unsaturated lower alkyl group includes lower alkenyl groups having about 2 to 6 carbon atoms, such as vinyl, allyl, 2-butenyl and 3-butenyl.

The aralkyl group represented by $R^4$ includes phenyl-lower($C_{1-6}$)alkyl groups, such as benzyl, phenethyl, phenylpropyl, phenylbutyl, $\alpha$-methylphenethyl and $\beta$-methylphenethyl.

In cases in which X is an optionally substituted imino group, X includes, for example, groups represented by the formula:

$$\underset{\displaystyle R^8}{\underset{|}{-N-}} \qquad (iii)$$

[wherein $R^8$ is hydrogen, optionally substituted alkyl, acyl or optionally substituted carbamoyl]. In the above formula (iii), the alkyl group represented by $R^8$ includes lower alkyl groups having about 1 to 5 carbon atoms, such as methyl, ethyl, propyl, butyl and pentyl, wherein the said alkyl groups may be substituted, for example, by carboxyl and lower($C_{1-5}$)alkoxycarbonyl (e.g., methoxycarbonyl, ethoxycarbonyl, propoxycarbonyl, butoxycarbonyl, pentoxycarbonyl, etc.)

The acyl group represented by $R^8$ includes, for example, lower alkanoyl having about 1 to 5 carbon atoms (e.g., formyl, acetyl, propionyl, butyryl, isobutyryl, valeryl, isovarelyl, etc.), benzoyl, phenoxycarbonyl, lower($C_{1-5}$)alkoxycarbonyl (e.g., methoxycarbonyl, ethoxycarbonyl, propoxycarbonyl, butoxycarbonyl, pentoxycarbonyl, etc.).

The optionally substituted carbamoyl group represented by $R^8$ includes, for example, carbamoyl, lower ($C_{1-5}$)alkylcarbamoyl (e.g., methylcarbamoyl, ethylcarbamoyl, propylcarbamoyl, butylcarbamoyl, etc.), di-lower($C_{1-5}$)alkylcarbamoyl (e.g., dimethylcarbamoyl, methylethylcarbamoyl, diethylcarbamoyl, methylpropylcarbamoyl, etc.), 3- to 7-membered cyclic aminocarbonyl [e.g., (aziridin-1-yl)carbonyl, (azetidin-1-yl)-carbonyl, (pyrrolidin-1-yl)carbonyl, piperidinocarbonyl, (perhydroazepin-1-yl)carbonyl, (piperazin-1-yl)-carbonyl, morphlinocarbonyl, thiomorpholinocarbonyl, etc.], and the like.

In cases in which Y is an optionally substituted imino group, Y includes, for example, groups represented by the formula:

$$\underset{\displaystyle R^9}{\underset{|}{-N-}} \qquad (iv)$$

In the formula (iv), the group represented by $R^9$ includes groups like those exemplified for $R^8$.

In cases in which X and Y both are an imino group, $R^8$ and $R^9$ may be the same or different, and $R^8$ and $R^9$ may combine with each other to form alkenylene, alkylene, etc.

The alkenylene and alkylene bridges which $R^8$ and $R^9$ combine with each other to form include lower alkenylene and alkylene bridges having about 1 to 4 carbon atoms, such as methylene, ethylene,

trimethylene, tetramethylene, vinylene and propenylene, and may be substituted by, for example, one or two oxo groups at their positions susceptible to substitution. The substituted alkylene and alkenylene include, for example, 1-oxoethylene, 3-oxopropenylene and 1,2-dioxoethylene. Specifically, the group represented by the formula:

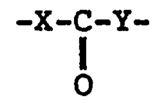

includes;

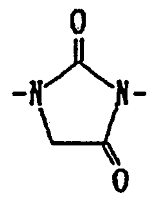 , 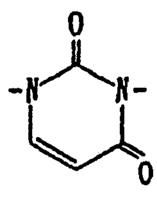 , 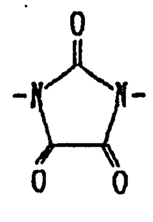 , 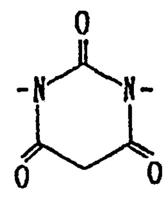

and the like.

In cases in which Y is an imino group, $R^9$ and $R^4$ may combine with each other to form alkenylene and alkylene, whereby the alkenylene and alkylene bridges which $R^9$ and $R^4$ combine with each other to form include lower alkenylene and alkylene bridges having about 1 to 4 carbon atoms, such as methylene, ethylene, trimethylene, tetramethylene, vinylene and propenylene, and these groups may be substituted by, for example, one or two oxo groups at their positions susceptible to substitution. The substituted alkylene and alkenylene groups include, for example, 1-oxoethylene, 3-oxopropenylene and 1,2-dioxoethylene. Specifically, the group represented by the formula:

$-Y-R^3-Z-R^4$

includes:

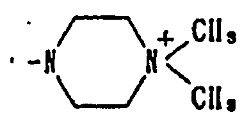

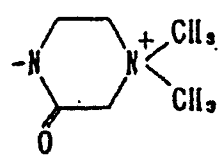

and the like.

As X, is preferable O.

Y includes preferably an imino group, which may be substituted more preferably a substituted imino group, still more preferably an imino group substituted by lower alkanoyl.

The optionally substituted imino group represented by Z includes imino groups substituted by a lower alkyl group having about 1 to 6 carbon atoms which may be substituted by lower($C_{1-4}$)alkoxycarbonyl (e.g., methylimino, ethylimino, propylimino, isopropylimino, butylimino, isobutylimino, sec-butylimino, tert-butylimino, pentylimino, hexylimino, ethoxycarbonylmethylimino, etc.), and imino groups substituted by aralkyl groups such as phenyl-lower($C_{1-6}$)alkyl (e.g., benzylimino, phenethylimino, phenylpropylimino, phenylbutylimino, ($\alpha$-methylphenethyl)imino, ($\beta$-methylphenethyl)imino, etc.).

The group Z-$R^4$ which $R^4$ and an optionally substituted imino group represented by Z form includes, for example, amino, N-[lower($C_{1-6}$)alkyl]amino, N,N-[di-lower($C_{1-6}$)alkyl]amino, N-[lower($C_{1-6}$)alkyl]-N-aralkylamino, N-aralkylamino, N,N-[di-lower($C_{1-6}$)alkyl]amino and the like. The above-mentioned aralkyl group includes phenyl-lower($C_{1-6}$)alkyl groups like those exemplified for $R^4$. The above-mentioned lower alkyl group may be substituted by, for example, lower($C_{1-4}$)alkoxycarbonyl, and may have an unsaturated bond [e.g., lower($C_{2-6}$)alkenyl]. Also, the imino group represented by Z may be converted into a quaternary salt to form the iminio group, and the said iminio group may be substituted by optionally lower($C_{1-4}$)-alkoxycarbonyl or carboxylato substituted lower alkyl having about 1 to 6 carbon atoms or aralkyl (e.g., phenyl-lower($C_{1-6}$)alkyl, etc.). The said substituted iminio group includes, for example, dimethyliminio, methylethyliminio, methylpropyliminio, methylbutyliminio, methylpentyliminio, methylhexylimino, diethyliminio, ethylpropyliminio, ethylbutyliminio, ethylpentyliminio, ethylhexyliminio, dipropyliminio, propyl-

butyliminio, propylpentyliminio, propylhexyliminio, dibutyliminio, butylpentyliminio, butylhexyliminio, dipentyliminio, pentylhexyliminio, dihexyliminio, benzylmethyliminio, dibenzyliminio, phenethylmethyliminio, diphenethyliminio, N-ethoxycarbonylmethyl-N-methyliminio and N-carboxylatomethyl-N-methyliminio.

The group Z-$R^4$ which $R^4$ and a substituted iminio group represented by Z form includes, for example, N,N,N-[tri-lower($C_{1-6}$)alkyl]ammonio, N,N-[di-lower($C_{1-6}$)alkyl]-N-aralkylammonio, N-[lower($C_{1-6}$)alkyl]-N,N-di-aralkylammonio, N,N,N-tri-aralkylammonio and the like. The above-mentioned aralkyl group includes phenyl($C_{1-6}$)alkyl groups like those exemplified for $R^4$. The above-mentioned lower alkyl group may be substituted by, for example, lower($C_{1-4}$)alkoxycarbonyl or carboxylato, and may have an unsaturated bond [e.g. lower($C_{2-6}$)alkenyl]. Among others, the compounds wherein Z is dimethyliminio and $R^4$ is methyl, i.e. those wherein Z-$R^4$ is trimethylammonio are preferable.

The nitrogen-containing heterocyclic ring represented by Z includes heterocyclic rings containing at least one nitrogen atom, such as monocyclic or bicyclic heterocyclic rings which may contain, as a ring forming atom, a nitrogen, oxygen or sulfur atom in addition to the said nitrogen atom. The said heterocyclic rings may be any of a saturated one, a partially saturated one or such minimum hydrogenated one as heteroaromatic rings, and their examples include azetidinyl, pyrrolidinyl, piperidinyl, perhydroazepinyl, pyrrolinyl, pyrazolinyl, pyrrolyl, pyridyl, oxazolyl, thiazolyl, pyridazinyl, pyrimidyl, pyrazinyl, imidazolyl, morpholinyl, thiomorpholinyl, piperazinyl, pyrazolidinyl, indolyl, isoindolyl, 1H-indazolyl, purinyl, quinolinyl, isoquinolinyl, 1,2,3,4-tetrahydroquinolinyl, perhydroindolyl and perhydroisoquinolinyl; in the case of monocyclic heterocyclic rings, among others, the 4- to 7-membered rings are preferable, and 5- to 6-membered rings are more preferable and thiazolyl or pyridyl is most preferable. These groups may be substituted at their positions susceptible to substitution by, for example, lower($C_{1-4}$)alkyl (e.g., methyl, ethyl, propyl, butyl, etc.), hydroxyl, amino(imino), mono- or di-lower($C_{1-4}$)alkylamino (e.g., methylamino, dimethylamino, etc.), carbamoyl, ureido, hydroxy- or amino-substituted lower($C_{1-4}$)alkyl (e.g., hydroxyethyl, aminoethyl, etc.), carboxyl, carboxylato and lower($C_{1-4}$)alkoxycarbonyl (e.g. methoxycarbonyl), and their examples include N-methylmorpholinyl, N-methylpiperidinyl, N-methylpiperazinyl, N-methylpyrrolidinyl, N-ethylpyrrolidinyl and the like.

The nitrogen atom in the said nitrogen-containing heterocyclic ring may be converted into a quaternary salt with $R^4$, and there may be mentioned, for example, groups, such as N,N-dimethylpyrrolidinio, N-methylpyridinio, N-ethylpyridinio, N-propylpyridinio, N-butylpyridinio, N-methyl-N-ethylpyrrolidinio, 3-methylthiazolio, 3-ethylthiazolio, 3-propylthiazolio, 3-butylthiazolio, N-allylpyridinio, N-ethoxycarbonylmethylpyridinio. Also, the said nitrogen atom may be converted into a quaternary salt by combining with $R^3$, and the heterocyclic ring containing the said nitrogen atom converted into a quaternary salt includes such groups as pyridinio-1-yl, oxazolio-3-yl, thiazolio-3-yl, pyridazinio-1-yl, pyrimidinio-1-yl, pyradinio-1-yl, quinolinio-1-yl, isoquinolinio-2-yl, 4-methylmorpholinio-4-yl, 1-methylpiperidinio-1-yl, 1-methylpiperazinio-1-yl, 1-methylpyrrolidinio-1-yl, 1- ethylpyrrolidinio-1-yl, 1-methylimidazolio-yl, 3-carboxylatopyridinio-1-yl, 3-methoxycarbonylpyridinio-1-yl and 4-dimethylaminopyridinio-1-yl.

In the case where $R^4$ is lower($C_{1-6}$)alkyl, lower($C_{1-4}$)alkoxycarbonyl-lower($C_{1-6}$)alkyl, carboxylato-lower($C_{1-4}$)alkyl or aralkyl, the position in the nitrogen-containing heterocyclic ring represented by Z to which $R^4$ is bound may be any position if it is a positions susceptible to such binding, but the case where the position in said heterocyclic ring to which $R^4$ is bound is the nitrogen atom is preferable in the case where the position in the said heterocyclic ring to which $R^3$ is bound is other than said nitrogen atom. In the case where the position in said heterocyclic ring to which $R^3$ is bound is the nitrogen atom, the position in said heterocyclic ring to which $R^4$ is bound may be any position if it is a position susceptible to such binding.

The group represented by Z-$R^4$ includes, for example, optionally lower($C_{1-6}$)alkyl ($C_{2-6}$ alkenyl), lower-($C_{1-4}$)alkoxycarbonyl-lower($C_{1-6}$)alkyl ($C_{2-6}$ alkenyl), carboxylato-lower($C_{1-6}$)alkyl ($C_{2-6}$ alkenyl) or aralkyl substituted azetidinyl, pyrrolidinyl, piperidinyl, perhydroazepinyl, pyrrolinyl, pyrazolinyl, pyrrolyl, pyridyl, oxazolyl, thiazolyl, pyridazinyl, pyrimidyl, pyradinyl, imidazolyl, morpholinyl, thiomorpholinyl, piperazinyl, pyrazolidinyl, indolyl, isoindolyl, 1H-indazolyl, purinyl, quinolinyl, isoquinolinyl, perhydroindolyl, perhydroisoquinolinyl, imidazolio, pyridinio, oxazolio, thiazolio, pyridazinio, pyrimidinio, pyradinio, quinolinio, isoquinolinio, morpholinio, piperidinio, piperazinio and pyrrolidinio groups. These groups may be further substituted by, for example, optionally hydroxy or amino substituted lower($C_{1-4}$))alkyl, hydroxy, amino-(imino), mono- or di-lower($C_{1-4}$)alkylamino, carbamoyl, ureido, carboxy, carboxylato or lower($C_{1-4}$)-alkoxycarbonyl.

With reference to the example of $R^4$ combining with the imino group represented by Y, specifically, the group as represented by the formula:

-Y-$R^3$-Z-$R^4$

includes;

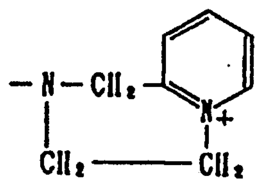,
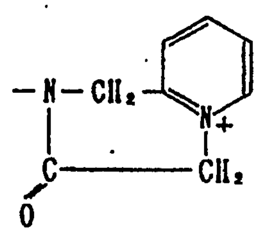

and the like.

As the nitrogen-containing heterocyclic ring represented by Z, the heterocyclic rings containing the quaternary nitrogen atom are more preferable.

The position in the heterocyclic ring to which $R^3$ is bound may be any position, only if it is a positions susceptible to such binding [e.g. 2-pyridyl, thiazol-2-yl, thiazol-4-yl, N-methylpyridinio-2-yl, N-methylpyridinio-3-yl, N-ethylpyridinio-2-yl, N-butylpyridinio-2-yl, N-methoxycarbonylmethylpyridinio-2-yl, N-ethylpyrrolidin-2-yl, N-methyl-N-ethylpyrrolidinio-2-yl, N-ethylpiperidin-3-yl, N-methyl-N-ethylpyperidinio-3-yl, 3-methylthiazolio-2-yl, 3-ethylthiazolio-2-yl, 3-propylthiazolio-2-yl, 3-butylthiazolio-2-yl, 3-methylthiazolio-4-yl, 3-ethylthiazolio-4-yl, 3,4-dimethylthiazolio-5-yl, N-allylpyridinio-2-yl], but the nitrogen atom or its adjacent positions (the positions adjacent to the nitrogen atom) are more preferable.

The salts of the compounds [I] include pharmaceutically acceptable salts, such as hydrochlorides, hydrobromides, hydroiodides, sulfates, nitrates and phosphates, and among others, the acid additions salts are preferable. In cases in which Z has the quaternary nitrogen atom, the compounds [I] may form salts with pharmaceutically acceptable anions such as acid anions (e.g. chlorine ion, bromine ion, iodine ion, sulfate ion, nitrate ion, phosphate ion and acetate ion) and hydroxyl ion, and may also form intramolecular salts.

The said salts with pharmaceutically acceptable anions and intramolecular salts fall within the scope of the pharmaceutically acceptable salt.

The compounds [I], in some instances, may have an asymmetric carbon in the molecule, depending upon the type of the substituent represented by $R^2$, and when the compound exists in two kinds of the stereoisomers with the R- and S-configurations, their individual isomers and mixture thereof both fall within the scope of the present invention.

The compounds [I] and salts thereof are known as PAF antagonists, and can be synthesized in the same methods as, or in similar methods to, those disclosed in literature references [e.g. Japanese Unexamined Patent Publication No. 243047/1985].

As a representative compound, there is mentioned CV-6209 which is disclosed in J. Pharmacol. Exp. Therap., 242, 263 (1987).

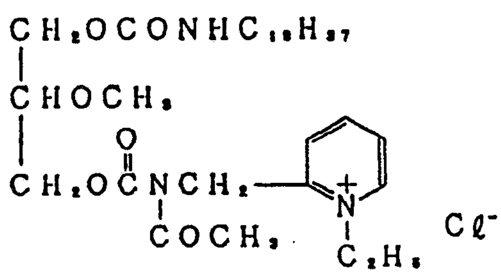

[II]

(CV-6209)

2a) The following compounds disclosed in the specification of Japanese Patent Application No. 64884/1988 (filed March 17, 1988) or European Patent Publication EP-A-0284359.

Compounds of the formula:

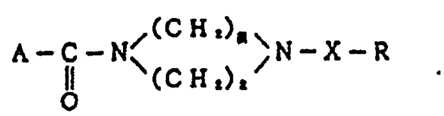

[IIIa]

[wherein A stands for a condensed polycyclic hydrocarbon group, R stands for a phenyl group substituted with a lower alkoxy group, X stands for methylene group, carbonyl group or thiocarbonyl group, and m denotes 2 or 3] and their salts.

Referring to the formula [IIIa], examples of the condensed polycyclic hydrocarbon groups shown by A include bicyclic or tricyclic hydrocarbon groups which may optionally be saturated partially, more specifically, hydrocarbon groups formed by condensation of two or three 5- to 8-membered rings, such as pentalenyl, indenyl (1H-indenyl, 2H-indenyl), indanyl, naphthyl, dihydronaphthyl (1,2-dihydronaphthyl, 2,3-dihydronaphthyl), tetrahydronaphthyl (1,2,3,4-tetrahydronaphthyl etc.), hexahydronaphthyl (1,2,3,4,5,6-hexahydronaphthyl etc.), azulenyl, heptalenyl, biphenylenyl, indacenyl (as-indacenyl, s-indacenyl), acenaphthylenyl, acenaphthenyl, phenalenyl, phenanthryl, dihydrophenanthryl (1,2-dihydrophenanthryl), tetrahydrophenanthryl (1,2,3,4-tetrahydrophenanthryl, etc.), hexahydrophenanthryl, anthryl, dihydroanthryl (9,10-dihydroanthryl, etc.), tetrahydroanthryl, hexahydroanthryl, octahydroanthryl, fluorenyl (3H-fluorenyl, 9H-fluorenyl, etc.), dihydrofluorenyl, tetrahydrofluorenyl, benzocycloheptenyl (5H-benzocycloheptenyl, etc.), dihydrobenzocycloheptenyl (6,7-dihydro-5H-benzocycloheptenyl, etc.), tetrahydrobenzocycloheptenyl (6,7,8,9-tetrahydro-5H-benzocycloheptenyl, etc.), dibenzocycloheptenyl (5H-dibenzo[a,b]cycloheptenyl, 5H-dibenzo[a,c]cycloheptenyl, etc.), naphthocycloheptenyl (6H-naphtho[b]cycloheptenyl, etc.), dihydronaphthocycloheptenyl (7,8-dihydro-6H-naphtho[b]cycloheptenyl, etc.), benzocyclooctenyl, dihydrobenzocyclooctenyl (5,6-dihydrobenzocyclooctenyl, etc.), tetrahydrobenzocyclooctenyl (5,6,7,8-tetrahydrobenzocyclooctenyl, etc.), hexahydrobenzocyclooctenyl, octahydrobenzocyclooctenyl, etc.

The condensed polycyclic hydrocarbon groups shown by the above A include, as a matter of course, those having one or more (preferably not more than 4) substituents such as, among others, a lower alkyl group, a halo lower alkyl group, a hydroxy lower alkyl group, an acyloxy lower alkyl group, a lower alkoxy-lower alkyl group, a lower alkoxy group, a halo lower alkoxy group, a lower alkoxy carbonyl-lower alkoxy group, a lower alkenyloxy group, aralkyloxy group, a lower alkoxy-lower alkoxy group, a lower alkoxycarbonyl group, carboxyl group, carbamoyl group, an N,N-di-lower alkylcarbamoyl group, an N-lower alkyl carbamoyl group, halo group, cyano group, nitro group, hydroxy group, acyloxy group, amino group, a lower alkylsulfonylamino group, acylamino group, a lower alkoxycarbonylamino group, acyl group, mercapto group, a lower alkylthio group, a lower alkylsulfinyl group, a lower alkylsulfonyl group and oxo group. When the condensed polycyclic hydrocarbon group has two or more substituents, the kinds of these substituents may be the same as or different from one another.

Lower alkyl groups as the above-mentioned substituents are exemplified by alkyl groups whose carbon number ranges from about 1 to about 4, such as methyl, ethyl, propyl, isopropyl, butyl, isobutyl, sec-butyl, tert-butyl, etc. As the halo lower alkyl group, mention is made of alkyl groups whose carbon number ranges from about 1 to about 4, which are substituted with 1 to 3 halo groups such as trifluoromethyl, fluoromethyl, chloromethyl, chloroethyl, fluoroethyl, etc. As the hydroxy lower alkyl group, mention is made of hydroxy alkyl groups whose carbon number ranges from about 1 to about 4, such as hydroxymethyl, hydroxyethyl, hydroxypropyl, hydroxybutyl, etc. As the acyloxy lower alkyl group, mention is made of alkyl groups whose carbon number ranges from about 1 to about 4, which are substituted with, for example, a lower alkanoyloxy group whose carbon number ranges from about 2 to about 5 such as acetoxyethyl, etc. or a benzoyloxy group such as benzoyloxyethyl, etc. As the lower alkoxy-lower alkyl group, mention is made of alkyl groups whose carbon number ranges from about 1 to about 4, which are substituted with, for example, an alkoxy group whose carbon number ranges from about 1 to about 4 such as methoxyethyl, ethoxyethyl, propoxyethyl, butoxyethyl, methoxypropyl, methoxybutyl, ethoxypropyl, ethoxybutyl etc. As the lower alkoxy group, mention is made of alkoxy groups whose carbon number ranges from about 1 to about 4 such as methoxy, ethoxy, propoxy, isopropoxy, butoxy, isobutoxy, sec-butoxy, tert-butoxy, etc. As the halo lower alkoxy group, mention is made of alkoxy groups whose carbon number ranges from about 1 to about 4, which are substituted with 1 to 3 halo groups such as chloroethoxy, fluoroethoxy, difluoroethoxy, trifluoroethoxy, chloropropoxy, chlorobutoxy, etc. As the lower alkoxy carbonyl-lower alkoxy group, mention is made of alkoxy groups whose carbon number ranges from about 1 to about 4, which are substituted with an alkoxycarbonyl group, the carbon number of the alkoxy moiety of which ranges from about 1 to about 4, such as methoxycarbonylmethoxy, ethoxycarbonylmethoxy, butoxycarbonylmethoxy, methoxycarbonylpropoxy, ethoxycarbonylethoxy, etc. Examples of the lower alkenyloxy group include alkenyloxy groups

whose carbon number ranges from about 2 to about 5, such as vinyloxy, allyloxy, butenyloxy, etc. As the aralkyloxy group, mention is made of phenyl lower alkyloxy groups, the carbon number of the lower alkyl moiety of which ranges from about 1 to about 4, such as benzyloxy, phenethyloxy, 3-phenylpropyloxy, α-methylphenethyloxy, α-methylbenzyloxy, α-ethylbenzyloxy, β-ethylphenethyloxy, β-methylphenethyloxy, etc. As the lower alkoxy-lower alkoxy group, mention is made of alkoxy groups whose carbon number ranges from about 1 to about 4, which are substituted with, for example, an alkoxy group whose carbon number ranges from about 1 to about 4, such as ethoxymethoxy, methoxyethoxy, butoxyethoxy, ethoxypropoxy, etc. Examples of the lower alkoxycarbonyl group include alkoxycarbonyl groups, the carbon number of the alkoxy moiety of which ranges from about 1 to about 4, such as methoxycarbonyl, ethoxycarbonyl, propoxycarbonyl, butoxycarbonyl, etc. As the N,N-di-lower alkylcarbamoyl group, mention is made of N,N-dialkylcarbamoyl groups, the carbon number of each alkyl moiety of which ranges from about 1 to about 4, such as N,N-dimethylcarbamoyl, N,N-diethylcarbamoyl, N,N-dipropylcarbamoyl, N,N-dibutylcarbamoyl, N-ethyl-N-methylcarbamoyl, etc., and groups forming 5- or 6-membered ring structure (e.g. pyrrolidinylcarbonyl, piperidinocarbonyl) by combining dialkyl moieties together. As the N-lower alkylcarbamoyl group, mention is made of N-alkylcarbamoyl groups, the carbon number of the alkyl moiety of which ranges from about 1 to about 4, such as N-methylcarbamoyl, N-ethylcarbamoyl, N-propylcarbamoyl, N-butylcarbamoyl, etc. As the halo group, mention is made of halogeno groups such as choro, fluoro, bromo, iodo, etc. As the acyloxy group, mention is made of alkanoyloxy groups, the carbon number of which ranges from about 2 to about 5, such as acetoxy, propanoyloxy, butyryloxy, pivaloyloxy, etc., and benzoyloxy group. As the lower alkylsulfonylamino group, mention is made of alkylsulfonylamino groups, the carbon number of which ranges from about 1 to about 4, such as methanesulfonylamino, ethanesulfonylamino, etc. Examples of the acylamino group include alkanoylamino groups, whose carbon number ranges from about 2 to about 5, such as acetylamino, propanoylamino, butyrylamino, pivaloylamino, etc. and benzoylamino. As the alkoxycarbonylamino group, mention is made of alkoxycarbonylamino groups, the carbon number of the alkoxy moiety of which ranges from about 1 to about 4, such as methoxycarbonylamino, ethoxycarbonylamino, propoxycarbonylamino, butoxycarbonylamino, etc. As the acyl group, mention is made of alkanoyl groups, the carbon number of which ranges from about 2 to about 5, such as acetyl, propanoyl, butyryl, pivaloyl, etc., and benzoyl group. As the lower alkylthio group, mention is made of alkylthio groups, the carbon number of which ranges from about 1 to about 4, such as methylthio, ethylthio, propylthio, butylthio, etc. As the lower alkylsulfinyl group, mention is made of alkylsulfinyl groups, the carbon number of which ranges from about 1 to about 4, such as methylsulfinyl, ethylsulfinyl, propylsulfinyl, butylsulfinyl, etc. As the lower alkylsulfonyl group, mention is made of alkylsulfonyl groups, the carbon number of which ranges from about 1 to about 4, such as methylsulfonyl, ethylsulfonyl, propylsulfonyl, butylsulfonyl, etc.

Specific examples of the condensed polycyclic hydrocarbon group shown by the above-mentioned A include 1-naphthyl, 2-naphthyl, 1-methoxy-2-naphthyl, 3-methoxy-2-naphthyl, 6-methoxy-2-naphthyl, 6,7-dimethoxy-2-naphthyl, 5,6,7-trimethoxy-2-naphthyl, 6-butoxy-2-naphthyl, 6,7-dibutoxy-2-naphthyl, 7-methoxy-1,2-dihydro-3-naphthyl, 6,7-dimethoxy-1,2-dihydro-3- naphthyl, 7,8-dimethoxy-1,2-dihydro-4-naphthyl, 6,7,8-trimethoxy-1,2-dihydro-3-naphthyl, 6,7-diethoxy-1,2-dihydro-3-naphthyl, 6,7-dipropoxy-1,2-dihydro-3-naphthyl, 6,7-dibutoxy-1,2-dihydro-3-naphthyl, 7-benzyloxy-1,2-dihydro-3-naphthyl, 7-hydroxy-1,2-dihydro-3-naphthyl, 6,7-dibenzyloxy-1,2-dihydro-3-naphthyl, 6,7-dihydroxy-1,2-dihydro-3-naphthyl, 6-methoxy-1,2,3,4-tetrahydro-2-naphthyl, 7-acetoxy-1,2-dihydro-3-naphthyl, 6,7-diacetoxy-1,2-dihydro-3-naphthyl, 7-benzoyloxy-1,2-dihydro-3-naphthyl, 6,7-dibenzoyloxy-1,2-dihydro-3-naphthyl, 7-methoxy-8-nitro-1,2-dihydro-3-naphthyl, 7-methoxy-6-nitro-1,2-dihydro-3-naphthyl, 6,7-dimethoxy-8-nitro-1,2-dihydro-3-naphthyl, 7-ethoxycarbonylmethoxy-1,2-dihydro-3-naphthyl, 7-(2-methoxyethoxy)-1,2-dihydro-3-naphthyl, 6,8-dimethyl-1,2-dihydro-3-naphthyl, 6-hydroxymethyl-7-methoxy-1,2-dihydro-3-naphthyl, 6,8-dimethyl-7-nitro-1,2-dihydro-3-naphthyl, 7-(2-hydroxyethoxy)-1,2-dihydro-3-naphthyl, 7-(2,3-dimethoxypropoxy)-1,2-dihydro-3-naphthyl, 7-(3-methoxypropoxy)-1,2-dihydro-3-naphthyl, 6,7-bis(2-methoxyethoxy)-1,2-dihydro-3-naphthyl, 5,6-dimethoxy-2-indanyl, 5,6-dimethoxy-1H-2-indenyl, 3,4-dimethoxy-6,7-dihydro-5H-8-benzocycloheptenyl, 2,3-dimethoxy-6,7-dihydro-5H-8-benzocycloheptenyl, 2,3-diethoxy-6,7-dihydro-5H-8-benzocycloheptenyl, 2,3-dipropoxy-6,7-dihydro-5H-8-benzocycloheptenyl, 2,3-dibutoxy-6,7-dihydro-5H-8-benzocycloheptenyl, 2-benzyloxy-3-methoxy-6,7-dihydro-5H-8-benzocycloheptenyl, 2-ethoxy-3-methoxy-6,7-dihydro-5H-8-benzocycloheptenyl, 2-propoxy-3-methoxy-6,7-dihydro-5H-8-benzocycloheptenyl, 2-butoxy-3-methoxy-6,7-dihydro-5H-8-benzocycloheptenyl, 3-methoxy-6,7-dihydro-5H-8-benzocycloheptenyl, 3-ethoxy-6,7-dihydro-5H-8-benzocycloheptenyl, 3-propoxy-6,7-dihydro-5H-8-benzocycloheptenyl, 3-butoxy-6,7-dihydro-5H-8-benzocycloheptenyl, 2,3-dimethyl-6,7-dihydro-5H-8- benzocycloheptenyl, 3-benzyloxy-6,7-dihydro-5H-8-benzocycloheptenyl, 6,7-dihydro-5H-8-benzocyclcheptenyl, 1,2,3-trimethoxy-6,7-dihydro-5H-8-benzocycloheptenyl, 7-ethoxy-1,2-dihydro-3-naphthyl, 7-propoxy-1,2-dihydro-3-naphthyl, 7-butoxy-1,2-dihydro-3-naphthyl, 2,3-dimethoxy-5,6,7,8-tetrahydro-9-benzocyclooctenyl, 5,6,7,8-tetrahydro-9-benzocyclooctenyl, 2,3-dihydro-3-naphthyl, 1-indanyl, 2-indanyl, 1H-2-indenyl, 2,3-dimethoxy-6,7,8,9-tetrahydro-5H-6-benzocycloheptenyl, 6,7-dimethoxy-1-hydroxy-2-naphthyl, 6-mercapto-2-naphthyl, 6-methylthio-2-naphthyl, 6-methanesulfonyl-2-naphthyl, 1-oxo-1,2,3,4-tetrahydro-6-naphthyl, 1-oxo-1,2,3,4-tetrahydro-7-naphthyl, 1-oxo-5-indanyl, 1-oxo-6-indanyl, 1-hydroxy-1,2,3,4-tetrahydro-6-naphthyl, 1-hydroxy-1,2,3,4-tetrahydro-7-naphthyl, 1-hydroxy-5-indanyl, 1-hydroxy-6-indanyl, 9-oxo-2-fluorenyl, 9-hydroxy-2-fluorenyl, 2-anthraquinonyl, etc.

As the A, condensed polycyclic hydrocarbon groups represented by the formula:

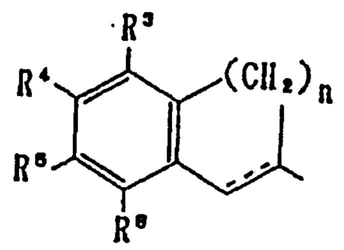

wherein the dotted line designates the presence or absence of double bond (more preferably the presence of double bond), n denotes an integer of 1 to 4 (more preferably 2 or 3), and $R^3$, $R^4$, $R^5$ and $R^6$ independently stand for hydrogen, a lower alkyl group, a halo lower alkyl group, a hydroxy lower alkyl group, an acyloxy lower alkyl group, a lower alkoxy-lower alkyl group, a lower alkoxy group, a halo lower alkoxy group, a lower alkoxy carbonyl-lower alkoxy group, a lower alkenyloxy group, aralkyloxy group, a lower alkoxy-lower alkoxy group, a lower alkoxycarbonyl group, carboxyl group, carbamoyl group, an N,N-di-lower alkylcarbamoyl group, an N-lower alkyl carbamoyl group, halo group, cyano group, nitro group, hydroxy group, acyloxy group, amino group, a lower alkylsulfonylamino group, acylamino group, a lower alkoxycarbonylamino group, acyl group, mercapto group, a lower alkylthio group, a lower alkylsulfinyl group or a lower alkylsulfonyl group (more preferably hydrogen, a lower alkoxy group, aralkyloxy group, a lower alkoxy-lower alkoxy group, hydroxy group or acyloxy group) are preferable and condensed polycyclic hydrocarbon groups represented by the above formula wherein $R^3$ and $R^6$ are hydrogen are more preferable.

As the A, condensed polycyclic hydrocarbon groups represented by the formula:

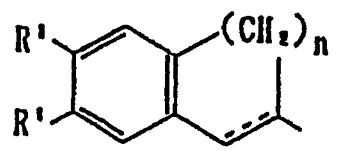

wherein the dotted line designates the presence or absence of double bond (more preferably the presence of double bond), $R^1$ stands for a lower alkoxy group (more preferably methoxy group or ethoxy group) and n denotes an integer of 1 to 4 (more preferably 2 or 3) are further preferable.

As the phenyl group substituted with a lower alkoxy group, represented by R, mention is made of, for example, phenyl groups substituted with one to five lower alkoxy groups, the carbon number of which ranges from about 1 to about 4, such as 2-methoxyphenyl, 3-methoxyphenyl, 4-methoxyphenyl, 2,3-dimethoxyphenyl, 2,4-dimethoxyphenyl, 2,5-dimethoxyphenyl, 2,6-dimethoxyphenyl, 3,4-dimethoxyphenyl, 3,5-dimethoxyphenyl, 3,4-diethoxyphenyl, 3,4-dipropoxyphenyl, 3,4-dibutoxyphenyl, 2,3,4- trimethoxyphenyl, 2,4,5-trimethoxyphenyl, 2,5,6-trimethoxyphenyl, 3,4,5-trimethoxyphenyl, 3,5-dimethoxy-4-ethoxyphenyl, 3,5-dimethoxy-4-propoxyphenyl, 3,5-dimethoxy-4-butoxyphenyl, 2,3,4,5-tetramethoxyphenyl, 2,3,4,5,6-pentamethoxyphenyl, etc.

As the R, phenyl groups substituted with three lower alkoxy groups are preferable, phenyl groups represented by the formula:

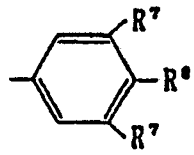

wherein $R^7$ and $R^8$ are independently methoxy group or ethoxy group are more preferable and phenyl groups represented by the above formula wherein at least one of $R^7$ and $R^8$ is methoxy group, and the other is methoxy group or ethoxy group are further preferable. Among others, a phenyl group represented by the formula:

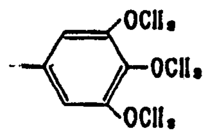

is most preferable.

X stands for methylene group ($-CH_2-$), carbonyl group

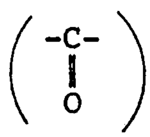

or thiocarbonyl group

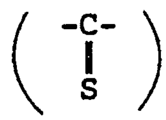

Depending on the value of m, the group

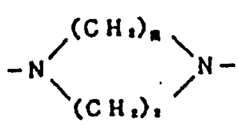

forms 1,4-piperazinediyl group (m = 2) or 1,4-homopiperazinediyl group (m = 3).
The value of m is preferably 2.

Among the compounds [IIIa], those wherein X is methylene may form salts with an inorganic acid such as hydrogen chloride, hydrobromic acid, sulfuric acid, nitric acid, phosphoric acid, etc. or an organic acid such as acetic acid, tartaric acid, citric acid, fumaric acid, maleic acid, toluenesulfonic acid, methanesulfonic acid, etc., or may form quaternary salts with a lower alkyl halide, the carbon number of the alkyl moiety of which ranges from about 1 to about 4 (e.g. methyl iodide, ethyl iodide, propyl iodide). As the salts of the compounds [IIIa], pharmacologically acceptable ones are preferable, and pharmacologically acceptable acid addition salts are more preferable. Hydrates of the compounds [IIIa] are also usable.

Among the above-mentioned compounds [IIIa], those represented by the formula:

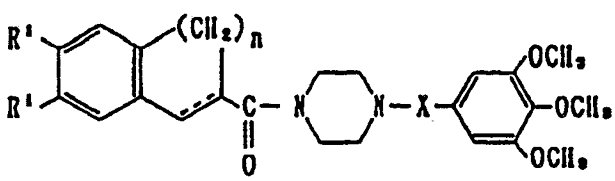 [IIIa']

wherein the dotted line designates the presence or absence of double bond (more preferably the presence of double bond), X is of the same meaning as defined above, $R^1$ stands for a lower alkoxy group (more preferably methoxy group or ethoxy group) and n denotes an integer of 1 to 4 (more preferably 2 or 3), and their pharmacologically acceptable acid addition salts (in case of X = methylene) are preferable.

The compounds [IIIa] and salts thereof can be synthesized, for example, in accordance with the methods disclosed in the specification of Japanese Patent Application No. 64884/1988 or European Patent Publication EP-A-0284359.

As a representative compound, there is mentioned:

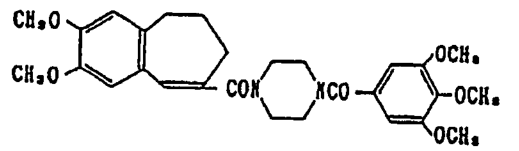

[IV]

2b) Compounds disclosed in the specification of Japanese Patent Application No. 296887/1987 (filed November 25, 1987), Japanese Patent Application No.295244/1988 (filed November 22, 1988) or European Patent Publication EP-A-0318235.

Compounds of the formula:

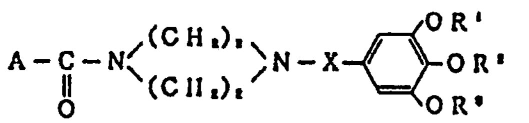

[IIIb]

[wherein A stands for an optionally substituted phenyl group or an optionally substituted heterocyclic group; X stands for a methylene group ($-CH_2-$), carbonyl group (-CO-) or thiocarbonyl group (-CS-); $R^1$, $R^2$ and $R^3$ each stand for a lower alkyl group] or salts thereof.

Referring to the formula [IIIb], examples of the heterocyclic groups shown by A include monocyclic, bicyclic or tricyclic heterocyclic groups which may optionally be saturated partially. Examples of the monocyclic heterocylic groups include 5- to 8-membered heterocyclic groups containing 1 to 3 hetero-atoms selected from nitrogen, oxygen and sulfur, more specifically, pyridyl (2-pyridyl, 3-pyridyl, 4-pyridyl), thienyl (2-thienyl, 3-thienyl), furyl, thiazolyl (2-thiazolyl, etc.) etc.

Examples of the bicyclic heterocyclic groups which may optionally be saturated partially include condensed bicyclic heterocyclic groups formed by condensation of a benzene ring and a 5- to 8-membered hetero-ring containing 1 to 3 hetero-atoms selected from nitrogen, oxygen and sulfur, more specifically, quinolyl (2-quinolyl, 3-quinolyl, 4-quinolyl, 5-quinolyl, 6-quinolyl, 7-quinolyl), isoquinolyl (3-isoquinolyl, etc.), indolyl (5-indolyl, etc.), benzothiazolyl (2-benzothiazolyl, etc. , 1,3-benzodioxolyl (1,3-benzodioxol-5-yl, etc.), benzofuranyl (2-benzofuranyl, etc.), 2,3-dihydrobenzofuranyl, benzopyranyl (2-benzopyranyl, 3-benzopyranyl, etc.), 3,4-dihydrobenzopyranyl, 1-benzoxepinyl(1-benzoxepin-4-yl, 1-benzoxepin-8-yl, etc.), 2,3-dihydro-1-benzoxepinyl(2,3-dihydro-1-benzoxepin-4-yl, etc.), 2,3,4,5-tetrahydro-1-benzoxepinyl, 1-benzothiepinyl, 2,3-dihydro-1-benzothiepinyl(2,3-dihydro-1-benzothiepin-4-yl, etc.), 2,3,4,5-tetrahydro-1-benzothiepinyl, 3,4-dihydro-2H-1,5-benzoxepinyl, 2,3-dihydro-1,4-benzodioxinyl[1,4-benzodioxanyl](2,3-dihydro-1,4-benzodioxin-6-yl, etc.), chromenyl(2H-chromen-3-yl, etc.), chromanyl(3-chromanyl, etc.), etc.

Examples of the tricyclic heterocyclic groups which may be saturated partially include condensed tricyclic heterocyclic groups formed by condensation of (i) 5- to 8-membered hetero-ring containing 1 to 3 hetero-atoms selected from nitrogen, oxygen and sulfur, (ii) benzene ring and (iii) benzene ring, 3- to 8-membered cycloalkane, 4- to 8-membered cycloalkene or 5- to 8-membered hetero-ring containing 1 to 3 hetero-atoms selected from nitrogen, oxygen and sulfur, and spiro-heterocyclic group formed by condensation of spiro-union of 3- to 8-membered cycloalkane to a condensed ring of (i) and (ii), etc., more specifically, dibenzofuranyl(2-dibenzofuranyl, etc.), 3,4-dihydrodibenzofuranyl(3,4-dihydrodibenzofuran-2-yl, etc.), 1,2,3,4-tetrahydrodibenzofuranyl, dibenzothiophenyl(2-dibenzothiophenyl, etc.), 3,4-dihydrodibenzothiophenyl(3,4-dihydrobenzothiophen-2-yl, etc.), 1,2,3,4-tetrahydrodibenzothiophenyl(1,2,3,4-tetrahydrobenzothiophen-2-yl, etc.), naphtho[2,3-d]-1,3-dioxolyl, 5,6-dihydronaphtho[2,3-d]-1,3-dioxolyl(5,6-dihydronaphtho[2,3-d]-1,3-dioxol-7-yl, etc.), 5,6,7,8-tetrahydronaphtho[2,3-d]-1,3-dioxolyl, naphtho[2,3-b]-1,4-dioxanyl, 6,7-dihydronaphtho[2,3-b]-1,4-dioxanyl(6,7-dihydronaphtho[2,3-b]-1,4-dioxan-8-yl, etc.), 5H-1,4-cyclohepta[f]-1,3-benzodioxolyl, 6,7-dihydro-5H-cyclohepta[f]-1,3-benzoxolyl(6,7-dihydro-5H-cyclohepta[f]-1,3-benzodioxol-8-yl, etc.), 6H- cyclohepta[g]-1,4-benzodioxanyl, 7,8-dihydro-6H-cyclohepta[g]-1,4-benzodioxanyl(7,8-dihydro-6H-cyclohepta[g]-1,4-benzodioxan-9-yl, etc.), dibenzo-p-dioxinyl, xanthenyl, 1,2-dihydroxanthenyl, naphtho[2,1-b]furanyl, 1,2,8,9-tetrahydronaphtho[2,1-b]furnayl, 2,3,5,6-tetrahydronaphtho-[2,1-b]furanyl, spiro[benzofuran-2(3H), 1′-cyclopropane]-yl(spiro[benzofuran-2(3H), 1′-cyclopropan]-5-yl, etc.), etc.

The phenyl group or heterocyclic groups shown by the above-mentioned A include those having one or more (preferably not more than 4) substituents such as, among others, a lower alkyl group, a halo lower alkyl group, a hydroxy lower alkyl group, an acyloxy lower alkyl group, a lower alkoxy lower alkyl group, a lower alkoxy group, a halo lower alkoxy group, a lower alkoxy carbonyl lower alkoxy group, a lower alkenyloxy group, aralkyloxy group, a lower alkoxy lower alkoxy group, a lower alkoxycarbonyl group, carboxyl group, carbamoyl group, an N,N-di-lower alkylcarbamoyl group, an N-lower alkyl carbamoyl group, halo group, cyano group, nitro group, hydroxy group, acyloxy group, amino group, a lower alkylsulfonylamino group, acylamino group, a lower alkoxycarbonylamino group, acyl group, mercapto group, a lower alkylthio group, a lower alkylsulfinyl group, a lower alkylsulfonyl group and oxo group. Among these substitutents, a lower alkyl group, a lower alkoxy group and oxo group are more preferable.

When the phenyl group or heterocyclic groups have two or more substituents, the kinds of these substituents may be the same or different from one another.

Lower alkyl groups as the above-mentioned substituents are exemplified by alkyl groups whose carbon number ranges from about 1 to about 4, such as methyl, ethyl, propyl, isopropyl, butyl, isobutyl, sec- butyl, tert-butyl, etc. As the halo lower alkyl group, mention is made of alkyl groups whose carbon number ranges from about 1 to about 4, which are substituted with 1 to 3 halo groups such as trifluoromethyl, fluoromethyl, chloromethyl, chloroethyl, fluoroethyl, etc. As the hydroxy lower alkyl group, mention is made of hydroxyalkyl groups whose carbon number ranges from about 1 to about 4, such as hydroxymethyl, hydroxyethyl, hydroxypropyl, hydroxybutyl, etc. As the acyloxy lower alkyl group, mention is made of alkyl groups whose carbon number ranges from about 1 to about 4, which are substituted with, for example, a lower alkanoyloxy group whose carbon number ranges from about 2 to about 5 or benzoyloxy group such as acetoxyethyl, benzoyloxyethyl, etc. As the lower alkoxy lower alkyl group, mention is made of alkyl groups whose carbon number ranges from about 1 to about 4, which are substituted with, for example, an alkoxy group whose carbon number ranges from about 1 to about 4 such as methoxyethyl, ethoxyethyl, propoxyethyl, butoxyethyl, methoxypropyl, ethoxypropyl, ethoxybutyl, etc. As the lower alkoxy group, mention is made of alkoxy groups whose carbon number ranges from about 1 to about 4 such as methoxy, ethoxy, propoxy, isopropoxy, butoxy, isobutoxy, sec-butoxy, tert-butoxy, etc. As the halo lower alkoxy group, mention is made of alkoxy groups whose carbon number ranges from about 1 to about 4, which are substituted with 1 to 3 halo groups such as chloroethoxy, fluoroethoxy, difluoroethoxy, trifluoroethoxy, chloropropoxy, chlorobutoxy, etc. As the lower alkoxy carbonyl lower alkoxy group, mention is made of alkoxy groups whose carbon number ranges from about 1 to about 4, which are substituted with an alkoxycarbonyl group, the carbon number of the alkoxy moiety of which ranges from about 1 to about 4, such as methoxycarbonylmethoxy, ethoxycarbonylmethoxy, butoxycarbonylmethoxy, methoxycarbonylpropoxy, ethoxycarbonylethoxy, etc. Examples of the lower alkenyloxy group include alkenyloxy groups whose carbon number ranges from about 2 to about 5, such as vinyloxy, allyloxy, butenyloxy, etc. As the aralkyloxy group, mention is made of phenyl lower alkyloxy groups, the carbon number of the lower alkyl moiety of which ranges from about 1 to about 4, such as benzyloxy, phenethyloxy, 3-phenylpropyloxy, α-methylphenethyloxy, α-methylbenzyloxy, α-ethylbenzyloxy, β-ethylphenethyloxy, β-methylphenethyloxy, etc. As the lower alkoxy lower alkoxy group, mention is made of alkoxy groups whose carbon number ranges from about 1 to about 4, which are substituted with, for example, an alkoxy group whose carbon number ranges from about 1 to about 4, such as ethoxymethoxy, methoxyethoxy, butoxyethoxy, ethoxypropoxy, etc. Examples of the lower alkoxycarbonyl group include alkoxycarbonyl groups, the carbon number of the alkoxy moiety of which ranges from about 1 to about 4, such as methoxycarbonyl, ethoxycarbonyl, propoxycarbonyl, butoxycarbonyl, etc. As the N,N-di-lower alkylcarbonyl group, mention is made of N,N-dialkylcarbamoyl groups, the carbon number of each alkyl moiety of which ranges from about 1 to about 4, such as N,N-dimethylcarbamoyl, N,N-diethylcarbamoyl, N,N-dipropylcarbamoyl, N,N-dibutylcarbamoyl, N-ethyl-N-methylcarbamoyl, etc., and groups forming 5- or 6-membered ring structure (e.g. N-pyrrolidinylcarbonyl, piperidinocarbonyl) by combining dialkyl moieties together. As the N-lower alkylcarbamoyl group, mention is made of N-alkylcarbamoyl groups, the carbon number of the alkyl moiety of which ranges from about 1 to about 4, such as N-methylcarbamoyl, N-ethylcarbamoyl, N-propylcarbamoyl, N-butylcarbamoyl,

etc. As the halo group, mention is made of halogeno groups such as chloro, fluoro, bromo, iodo, etc. As the acyloxy group, mention is made of alkanoyloxy groups, the carbon number of which ranges from about 2 to about 5, such as acetoxy, propanoyloxy, butyryloxy, pivaloyloxy, etc., and benzoyloxy group. As the lower alkylsulfonylamino group, mention is made of alkylsulfonylamino groups, the carbon number of which ranges from about 1 to about 4, such as methanesulfonylamino, ethanesulfonylamino, etc. Examples of the acylamino group include alkanoylamino groups, whose carbon number ranges from about 2 to about 5, such as acetamido, propanoylamino, butyrylamino, pivaloylamino, etc. and benzamido group. As the lower alkoxycarbonylamino group, mention is made of alkoxycarbonylamino groups, the carbon number of the alkoxy moiety of which ranges from about 1 to about 4, such as methoxycarbonylamino, ethoxycarbonylamino, propoxycarbonylamino, butoxycarbonylamino, etc. As the acyl group, mention is made of alkanoyl groups, the carbon number of which ranges from about 2 to about 5, such as acetyl, propanoyl, butyryl, pivaloyl, etc., and benzoyl group. As the lower alkylthio group, mention is made of alkylthio groups, the carbon number of which ranges from about 1 to about 4, such as methylthio, ethylthio, propylthio, butylthio, etc. As the lower alkylsulfinyl group, mention is made of alkylsulfinyl groups, the carbon number of which ranges from about 1 to about 4, such as methylsulfinyl, ethylsulfinyl, propylsulfinyl, butylsulfinyl, etc. As the lower alkylsulfonyl group, mention is made of alkylsulfonyl groups, the carbon number of which ranges from about 1 to about 4, such as methylsulfonyl, ethylsulfonyl, propylsulfonyl, butylsulfonyl, etc.

Specific examples of the substituted phenyl group shown by the above-mentioned A include 3,4-dimethoxyphenyl, 3,4,5-trimethoxyphenyl, 3,4,5-triethoxyphenyl, 3-methoxy-5-nitro-4-n-propoxyphenyl, 3,4-dimethoxy-5-methylsulfonylphenyl, 3-chloro-4,5-dimethoxyphenyl, etc. Examples of substituted monocyclic, dicyclic or tricyclic heterocyclic groups include 3-coumarinyl, 6-methoxy-3-coumarinyl, 7-methoxy-3-coumarinyl, 6-methyl-3-coumarinyl, 7-N-butoxy-coumarinyl, 6-methoxy-2-benzofuranyl, 7-ethoxy-2-benzofuranyl, 1-hydroxy-1,2,3,4-tetrahydrodibenzothiophen-2-yl, 3-oxospiro[benzofuran-2(3H), 1′-cyclopropan]-5-yl, 3-hydroxyspiro[benzofuran-2(3H), 1′-cyclopropan]-5-yl, 7,8-dimethoxy-2,3-dihydro-1-benzoxepin-4-yl, 7-methoxy-2,3-dihydro-1-benzoxepin-4-yl, 8-propoxy-2,3-dihydro-1-benzoxepin-4-yl, 7-methoxy-8-methyl-2,3-dihydro-1-benzoxepin-4-yl, 7,8-dimethoxy-2,3,4,5-tetrahydro-1-benzoxepin-4-yl, 7-chloro-8-methoxy-2,3-dihydro-1-benzoxepin-4-yl, 7,8-dimethyl-2,3-dihydro-1-benzoxepin-4-yl, 7,8-dimethoxy-2,3-dihydro-1-benzothiepin-4-yl, 8-methoxy-2,3-dihydro-1-benzothiepin-4-yl, 1-oxo-2,3-dihydro-1-benzothiepin-4-yl, 1,1-dioxo-2,3-dihydro-1-benzothiepin-4-yl, 7,8-dimethoxy-1-oxo-2,3-dihydro-1-benzothiepin-4-yl, 7,8-dimethoxy-1,1-dioxo-2,3-dihydro-1-benzothiepin-4-yl, etc.

Preferable examples of A include phenyl groups substituted with 2 to 3 lower alkoxy groups (more preferably methoxy group or ethoxy group) or optionally substituted oxygen-containing condensed dicyclic or tricyclic heterocyclic groups (benzo-1,3-dioxolyl, coumarinyl, 2,3-dihydro-1-benzoxepinyl, dibenzofuranyl, 5,6-dihydronaphtho[2,3-d]-1,3-dioxolyl, 6,7-dihydrocyclohepta[f]-1,3-benzodioxolyl, etc.).

Examples of the lower alkyl group shown by $R^1$, $R^2$ or $R^3$ include alkyl groups, the carbon number of which ranges from about 1 to about 4, such as methyl group, ethyl group, propyl group, etc. $R^1$, $R^2$ and $R^3$ may be the same or different from one another, while it is preferable that $R^1$, $R^2$ and $R^3$ are all methyl groups. As X, carbonyl group is preferable.

Among them, compounds of the formula :

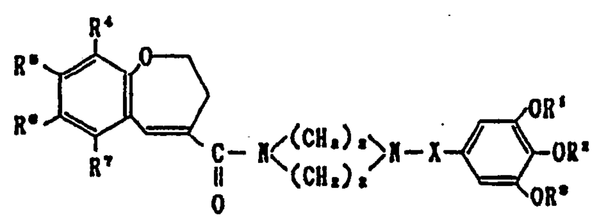

[IIIb′]

[wherein $R^1$, $R^2$, $R^3$ and X are of the same meaning as defined above; $R^4$, $R^5$, $R^6$ and $R^7$ each stand for hydrogen or a substituent of the phenyl group or heterocyclic group shown by the above-mentioned A] and salts thereof are preferable.

Among the compounds of the formula [IIIb′], those wherein $R^4$ and $R^7$ are hydrogen, and $R^5$ and $R^6$ are a lower alkoxy group (more preferably methoxy group) are further preferable.

Among the compounds of the formula [IIIb] and [IIIb′], those wherein X is methylene group may form salts with an inorganic acid such as hydrogen chloride, hydrogen bromide, sulfuric acid, nitric acid, phosphoric acid, etc. or an organic acid such as acetic acid, tartaric acid, citric acid, fumaric acid, maleic acid, toluenesulfonic acid, methanesulfonic acid, etc., or may form quaternary salts with a lower alkyl halide, the carbon number of the alkyl moiety of which ranges from about 1 to about 4 (e.g. methyl iodide, ethyl

iodide, propyl iodide). As the salts of the compounds [IIIb], pharmaceutically acceptable ones are preferable, and pharmaceutically acceptable acid addition salts are more preferable. Hydrates of the compounds [IIIb] are also usable.

The compounds [IIIb] and salts thereof can be synthesized, for example, in accordance with the methods disclosed in the specification of Japanese Patent Application No. 296887/1987, Japanese Patent Application No. 295244/1988 or European Patent Publication EP-A-0318235.

3) The following compounds disclosed in Japanese Unexamined Patent Publication No. 35116/1983.

Glycerol derivatives of the formula:

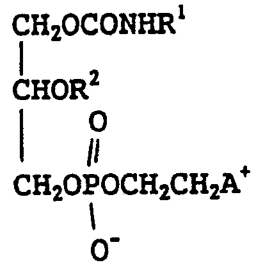

[V]

[wherein $R^1$ is a higher alkyl group, $R^2$ is a lower alkyl group and $A^+$ is a heterocyclic group containing a quaternized nitrogen atom] and salts thereof.

Referring to the above formula, the higher alkyl group represented by $R^1$ includes straight or branched chain alkyl groups having 10 to 24 carbon atoms, such as decyl, dodecyl, tridecyl, tetradecyl, pentadecyl, hexadecyl, heptadecyl, octadecyl, nonadecyl, eicosanyl, docosanyl, farnesyl and dihydrophytyl. $R^1$ is preferably an alkyl group having 14 to 20 carbon atoms and most preferably an n-octadecyl group.

The lower alkyl group represented by $R^2$ includes alkyl groups having 1 to 4 carbon atoms, such as methyl, ethyl, propyl, isopropyl, butyl and isobutyl, and desirably is a methyl group.

The heterocyclic group containing a quaternized nitrogen atom represented by $A^+$ includes, for example, pyridinio, oxazolio, isoxazolio, thiazolio, isothiazolio, pyridazinio, quinolinio and isoquinolinio. These heterocyclic groups may optionally have a substituent such as lower ($C_{1-4}$) alkyl (e.g. methyl, ethyl), hydroxy, hydroxyethyl, aminoethyl, amino (imino), carbamoyl or ureido. Among the above heterocyclic groups, preferred is a thiazolio group.

As the salts of compounds [V], there are mentioned pharmaceutically acceptable salts of the formula:

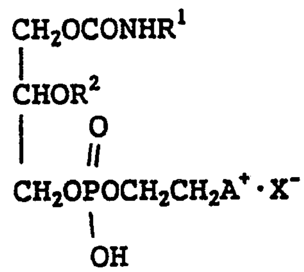

[Va]

[wherein $X^-$ is an anion such as chloro, bromo, iodo or tosylate ion and the other symbols are as defined above], or by the formula

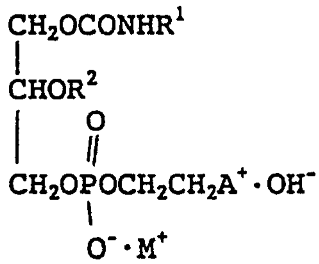

[Vb]

[wherein $M^+$ is an alkali metal (e.g. sodium, potassium) or alkaline earth metal (e.g. calcium, magnesium) ion and the other symbols are as defined above].

The compounds [V] and salts thereof are known as PAF antagonists [e.g. Japanese Unexamined Patent Publication No. 35116/1983; Life Sci., 32, 1975-1982(1983)], and can be synthesized in the same methods as, or in similar methods to, those disclosed in literature references [e.g. Japanese Unexamined Patent Publication No. 35116/1983, Japanese Unexamined Patent Publication No. 35194/1983, European Patent Publication EP-A-0035375].

As a representative compound, there is mentioned CV-3988 which is disclosed in Life Sci., 32, 1975-(1983).

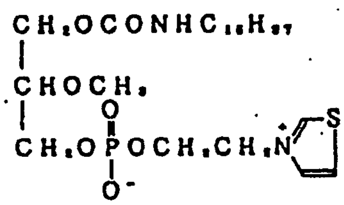

(CV-3988)

[VI]

4) Compounds disclosed in the specification of Japanese Patent Application No. 186494/1988 (filed July 25, 1988) or European Patent Publication EP-A-0301751.

Compounds of the formula:

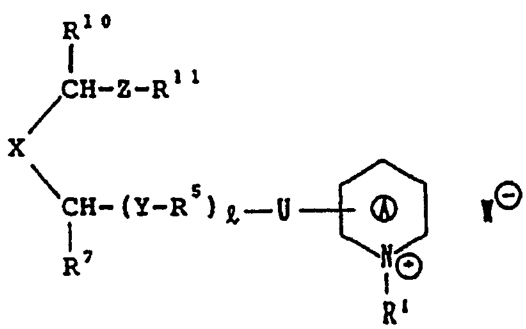

[VII]

[wherein

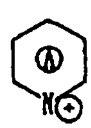

stands for an optionally substituted pyridinium ring; $R^1$ stands for a lower alkyl group or aralkyl group; $R^7$ and $R^{10}$ each stand for hydrogen, a lower alkyl group, aryl group or aralkyl group; $\ell$ denotes 0 or 1; $R^5$

stands for a phenylene group or an alkylene group which may be substituted; $R^{11}$ stands for an alkyl group or an aryl group; X stands for a group of the formula $-CH_2OCH_2-$ or a group of the formula

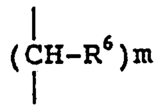

[wherein $R^6$ stands for hydrogen, a lower alkyl or a lower alkoxy, and m denotes an integer of 0 to 3); U stands for a group of the formula

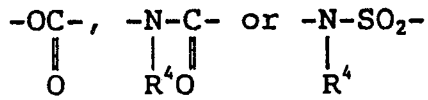

(wherein $R^4$ stands for hydrogen, a lower alkyl group, aryl group or aralkyl group); Y and Z each stand for a divalent chain group consisting of one to six members which is selected from the class consisting of groups of the formulae

-O-, - N - -CO-, -S- and $-SO_2-$
R

(wherein R stands for hydrogen, a lower alkyl group, acyl group or aryl group) and at least one of which is a group of the formula

-O- or - N -,
R

with the proviso that R may be the same or different from each other, or may form a ring together when two or more groups of the formula

- N -
R

are contained, that R may be bonded to $R^4$ when Y contains a group of the formula - N -
R

and that R may be bonded to $R^{11}$ when Z contains a group of the formula

- N - ;
R

and $W^{\theta}$ stands for a counter anion.

In the formula [VII], the group

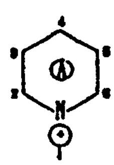

shows an optionally substituted pyridinium ring. To the 1-position of the pyridinium ring is bonded the group $R^1$, and to the 2- to 6-positions is attached one side chain represented by the following formula:

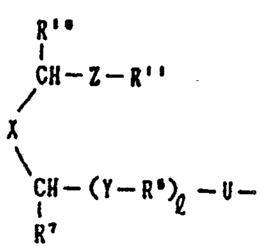

The pyridinium ring may have, at a position, other than its 1-position and the position to which the side chain is bonded, 1 to 4 (preferably 1 to 2) substituents such as a halogeno group, a lower alkyl group, a

lower alkoxy group, a nitro group, a cyano group, a lower alkoxycarbonyl group, a carbamoyl group, a lower alkyl carbamoyl group, etc. or may have an aromatic ring bonded thereto. The side chain is preferably bonded to the 2- to 4-position, and the substituents are preferably bonded to one or two of the 3- to 5-position of the pyridinium ring.

Examples of the lower alkyl group as $R^1$, $R^4$, $R^6$, $R^7$, $R^{10}$ or a substituent at the pyridinium ring include straight-chain or branched alkyl groups having 1 to 6 carbon atoms, such as methyl, ethyl, propyl, isopropyl, butyl, isobutyl, sec-butyl, tert-butyl, pentyl hexyl, etc. The lower alkyl groups may optionally have an unsaturated bond, which are exemplified by lower alkenyl groups having 2 to 6 carbon atoms, such as vinyl, allyl, 2-butenyl, 3-butenyl, etc.

Examples of the lower alkoxy group as $R^6$ or a substituent at the pyridinium ring include straight-chain or branched alkoxy groups having 1 to 6 carbon atoms, such as methoxy, ethoxy, propoxy, isopropoxy, butoxy, pentoxy, etc.

The lower alkoxy group as $R^6$ may have substituents, which may be combined together to form a 5- to 7-membered hetero ring (e.g., imidazolyl, oxazolyl, isoxazolyl, thiazolyl, etc.), and said hetero ring may have substituents such as lower alkyl group, acyl group, aryl group, aralkyl group, etc.

Examples of the halogeno group as a substituent at the pyridinium ring include fluoro, bromo, chloro, iodo, etc.

Examples of the lower alkoxycarbonyl group as a substituent at the pyridinium ring include alkoxycarbonyl groups whose alkoxy moiety has about 1 to about 6, such as methoxycarbonyl, ethoxycarbonyl, propoxycarbonyl, butoxycarbonyl, etc.

Examples of the lower alkylcarbamoyl group as a substituent at the pyridinium ring include N-alkyl carbamoyl groups whose alkyl moiety has about 1 to about 6, such as methyl carbamoyl, ethyl carbamoyl, propyl carbamoyl, butyl carbamoyl, etc., and N,N-dialkyl carbamoyl groups, the carbon number of each alkyl moiety being about 1 to about 6, such as dimethyl carbamoyl, diethyl carbamoyl, dibutyl carbamoyl, methyl ethyl carbamoyl, etc.

Examples of the aryl group as $R^4$, $R^7$ or $R^{10}$ include aromatic monocyclic, dicyclic or tricyclic hydrocarbon residue, such as phenyl, 1-naphthyl, 2-naphthyl, phenanthryl, anthryl, etc., or aromatic monocyclic or dicyclic heterocyclic ring such as thienyl, furyl, benzothienyl, benzofuranyl, etc. The aryl group may have 1 to 4 (preferably 1 to 2) substituents such as a halogeno group, a lower alkyl group, a lower alkoxy group, a nitro group, a cyano group, an oxo group, a hydroxy group, an amino group, a lower alkoxy carbonyl group, a carbamoyl group, a lower alkyl carbamoyl group, etc. Examples of the lower alkyl group include alkyl groups whose carbon number is about 1 to about 6, and the said lower alkyl groups may have an unsaturated bond. Examples of the lower alkyl groups include lower alkenyl groups whose carbon number ranges from about 2 to about 6. Examples of the alkyl groups whose carbon number ranges from about 1 to about 6 and of the lower alkenyl groups whose carbon number ranges from about 2 to about 6 include, as practical ones, lower alkyl groups mentioned as the substituents at the above-mentioned pyridinium ring. As the lower alkoxy group, mention is made of alkoxy groups whose carbon number ranges from about 1 to about 6. As the lower alkoxycarbonyl group, mention is made of alkoxycarbonyl group, the carbon number of the alkoxy moiety of which ranges from about 1 to about 6. As the lower alkylcarbamoyl group, mention is made of N- alkylcarbamoyl group, the carbon number of the alkyl moiety of which ranges from about 1 to about 6, and of N,N-dialkylcarbamoyl, the carbon number of each alkyl, moiety of which ranges from about 1 to about 6. Practical examples of these groups include groups similar to lower alkoxy groups, lower alkoxycarbonyl groups and lower alkylcarbamoyl groups set forth as substituents of the above-mentioned pyridinium ring. As the aryl group having an oxo group, mention is made of, for example, benzoquinonyl, naphthoquinonyl, anthraquinonyl, etc.

Examples of the aralkyl groups as $R^1$, $R^4$, $R^7$ and $R^{10}$ include phenyl-lower alkyl group, the carbon number of the alkyl moiety of which ranges from about 1 to about 6, and naphthyl-lower alkyl group, the carbon number of the alkyl moiety of which ranges from about 1 to about 6. The phenyl moiety of phenyl-lower alkyl groups and the naphthyl moiety of naphthyl-lower alkyl groups may have 1 to 4 (preferably 1 or 2) substituents such as halogeno group, a lower alkyl group, a lower alkoxy group, nitro group, cyano group, oxo group, hydroxyl group, amino group, a lower alkoxycarbonyl group, carbamoyl group, a lower alkylcarbamoyl group, etc. As these substituents, mention is made of groups similar to the above-mentioned substituents at the aryl groups.

Examples of the phenylene groups as $R^5$ include o-phenylene(1,2-phenylene), m-phenylene(1,3-phenylene) and p-phenylene(1,4-phenylene).

Examples of the alkylene groups as $R^5$ include alkylene groups whose carbon number ranges from about 1 to about 6, such as methylene, ethylene, trimethylene, tetramethylene, pentamethylene, hexamethylene, etc. Said alkylene groups may have substituents such as lower alkyl groups whose carbon

number ranges from about 1 to 5, etc.

The alkyl groups shown by $R^{11}$ are exemplified by straight-chain or branched alkyl groups whose carbon number ranges from about 1 to about 30 (preferably $C_{1-18}$), such as methyl, ethyl, propyl, butyl, pentyl, hexyl, heptyl, octyl, nonyl, decyl, undecyl, tridecyl, tetradecyl, pentadecyl, hexadecyl, heptadecyl, octadecyl, nonadecyl, eicosanyl, heneicosanyl, docosanyl, tricosanyl, tetracosanyl, pentacosanyl, hexacosanyl, heptacosanyl, octacosanyl, nonacosanyl, triacontanyl, farnesyl, dihydrophytyl, etc.; cycloalkyl groups whose carbon number ranges from about 3 to about 8, such as cyclopropyl, cyclobutyl, cyclopentyl, cycloheptyl, cyclooctyl, etc.; bicycloalkyl groups whose carbon number ranges from about 7 to about 12, such as norbornyl, bicyclo[2,2,2]octyl, bicyclo[3,3,1]nonyl, bicyclo[3,3,0]octyl, etc.; tricycloalkyl groups whose carbon number ranges from about 7 to about 12, such as adamantyl, etc.; bicyclic hydrocarbon residues formed by condensation of 5- to 8-membered ring, such as perhydropentalenyl, perhydroindenyl, perhydroazulenyl, perhydrocyclopentacyclooctenyl, perhydronaphthyl, perhydrobenzocycloheptenyl, perhydrobenzocyclooctenyl, perhydroheptalenyl, perhydrocycloheptacyclooctenyl, etc.; tricyclic hydrocarbon residues formed by condensation of 5- to 8-membered ring, such as perhydroindacenyl (asymmetric or symmetric) perhydroacenaphthylenyl, perhydrophenanthryl, perhydroanthryl, etc.; etc. The above-mentioned alkyl groups may optionally have unsaturated bond, and the unsaturated alkyl groups are exemplified by alkenyl groups whose carbon number ranges from about 2 to about 30, such as vinyl, allyl, 9-octadecenyl, etc.; cycloalkenyl groups whose carbon number ranges from about 5 to about 8, such as cyclopentenyl, cyclohexenyl, etc.; bicycloalkenyl groups whose carbon number ranges from about 7 to about 12, such as bicyclo[2,2,2]oct-2-enyl, etc.; tricycloalkenyl groups whose carbon number ranges from about 7 to about 12; bicyclic hydrocarbon residues formed by condensation of a benzene ring with 5- to 8-membered ring, such as indanyl(1-indanyl, 2-indanyl, etc.), indenyl(1H-inden-1-yl, 1H-inden-2-yl, 1H-inden-3-yl, etc.), dihydronaphthyl(1,2-dihydro-1-naphthyl, 1,2-dihydro-2-naphthyl, etc.), tetrahydronaphthyl(5,6,7,8-tetrahydro-1-naphthyl, 5,6,7,8-tetrahydro-2-naphthyl, etc.), 5H-benzocycloheptenyl(5H-5-benzocycloheptenyl, 5H-8-benzocycloheptenyl, etc.), dihydro-5H-benzocycloheptenyl(6,7-dihydro-5H-8-benzocycloheptenyl, etc., tetrahydrobenzocyclooctenyl(5,6,7,8-tetrahydro-9-benzocyclooctenyl, etc.); tricyclic hydrocarbon residue formed by condensation of two benzene rings with 5- to 8-membered ring, such as acenaphthenyl(1-acenaphthenyl, etc.), tetrahydroanthryl(1,2,3,4-tetrahydro-1-anthryl, etc.); etc.

The above-mentioned alkyl groups whose carbon number ranges from about 1 to about 30 and alkenyl groups whose carbon number ranges from about 2 to about 30 may have about one to about four (preferably one or two) substituents which are exemplified by cycloalkyl group, the carbon number of which ranges from 3 to about 8, phenyl group, naphthyl group, halogeno or a lower alkoxy group, whose carbon number ranges from about one to about six, etc. The phenyl groups as the substituents on the alkyl groups and the alkenyl groups may have about one to about four substituents which are exemplified by a lower alkyl group whose carbon number ranges from 1 to about 6, a lower alkoxy group whose carbon number ranges from about 1 to about 6, hydroxy group, nitro group, halogeno group, etc.

Cycloalkyl group, bicycloalkyl group, tricycloalkyl group, bicyclic hydrocarbon residue, tricyclic hydrocarbon residue as well as these groups having an unsaturated bond; which are included in the alkyl groups shown by $R^{11}$, may have about 1 to about 4 (preferably 1 or 2) substituents such as a lower alkyl group, a halogeno lower alkyl group, a hydroxy lower alkyl group, an acyloxy lower alkyl group, a lower alkoxy lower alkyl group, a lower alkoxy, a halogeno lower alkoxy group, a lower alkoxycarbonyl lower alkoxy group, a lower alkenyloxy group, aralkyloxy group, a lower alkoxy lower alkoxy group, a lower alkoxycarbonyl group, carboxyl group, carbamoyl group, an N,N-di-lower alkylcarbamoyl group, an N-lower alkyl carbamoyl group, halogeno group, cyano group, nitro group, hydroxyl group, acyloxy group, amino group, a lower alkylsulfonylamino group, acylamino group, a lower alkoxycarbonylamino group, acyl group, mercapto group, a lower alkylthio group, a lower alkyl sulfinyl group, a lower alkylsulfonyl group, oxo group, etc. When they have two or more substituents, the kinds of these substituents may be the same or different from one another.

Lower alkyl groups as the above-mentioned substituents are exemplified by alkyl groups whose carbon number ranges from about 1 to about 6, such as methyl, ethyl, propyl, isopropyl, butyl, isobutyl, sec-butyl, tert-butyl, etc. As the halogeno lower alkyl group, mention is made of alkyl groups whose carbon number ranges from about 1 to about 6, which are substituted with 1 to 3 halogeno groups, such as trifluoromethyl, fluoromethyl, chloromethyl, chloroethyl, fluoroethyl, etc. As the hydroxy lower alkyl group, mention is made of hydrocy alkyl groups whose carbon number ranges from about 1 to about 6, such as hydroxymethyl, hydroxyethyl, hydroxypropyl, hydroxybutyl, etc. As the acyloxy lower alkyl group, mention is made of alkyl groups whose carbon number ranges from about 1 to about 6, which are substituted with, for example, a lower alkanoyloxy group whose carbon number ranges from about 2 to about 6 or a benzoyloxy group such as acetoxyethyl, benzoyloxyethyl, etc. As the lower alkoxy-lower alkyl group, mention is made of alkyl

groups whose carbon number ranges from about 1 to about 6, which are substituted with, for example, an alkoxy group whose carbon number ranges from about 1 to about 6 such as methoxyethyl, ethoxyethyl, propoxyethyl, butoxyethyl, methoxypropyl, methoxybutyl, ethoxypropyl, ethoxybutyl, etc. As the lower alkoxy group, mention is made of alkoxy groups whose carbon number ranges from about 1 to about 6, such as methoxy, ethoxy, propoxy, isopropoxy, butoxy, isobutoxy, sec-butoxy, tert-butoxy, etc. As the halogeno lower alkoxy group, mention is made of alkoxy groups whose carbon number ranges from about 1 to about 6, which are substituted with 1 to 3 halogeno groups such as chloroethoxy, fluoroethoxy, difluoroethoxy, trifluoroethoxy, chloropropoxy, chlorobutoxy, etc. As the lower alkoxy carbonyl-lower alkoxy group, mention is made of alkoxy groups whose carbon number ranges from about 1 to about 6, which are substituted with an alkoxycarbonyl group, the carbon number of the alkoxy moiety of which ranges from about 1 to about 6, such as methoxycarbonylmethoxy, ethoxycarbonylmethoxy, butoxycarbonylmethoxy, methoxycarbonylpropoxy, ethoxycarbonylethoxy, etc. Examples of the lower alkenyloxy group include alkenyloxy groups whose carbon number ranges from about 2 to about 6, such as vinyloxy, allyloxy, butenyloxy, etc. As the aralkyloxy group, mention is made of phenyl lower alkyloxy groups, the carbon number of the lower alkyl moiety of which ranges from about 1 to about 6, such as benzyloxy, phenethyloxy, 3-phenylpropyloxy, α-methylphenethyloxy, α-methylbenzyloxy, α-ethylbenzyloxy, β-ethylphenethyloxy, β-methylphenethyloxy, etc. As the lower alkoxy-lower alkoxy group, mention is made of alkoxy groups whose carbon number ranges from about 1 to about 6, which are substituted with, for example, an alkoxy group whose carbon number ranges from about 1 to about 6, such as ethoxymethoxy, methoxyethoxy, butoxyethoxy, ethoxypropoxy, etc. Examples of the lower alkoxycarbonyl group include alkoxycarbonyl groups, the carbon number of the alkoxy moiety of which ranges from about 1 to about 6, such as methoxycarbonyl, ethoxycarbonyl, propoxycarbonyl, butoxycarbonyl, etc. As the N,N-di-lower alkylcarbamoyl group, mention is made of N,N-dialkylcarbamoyl group, the carbon number of each alkyl moiety of which ranges from about 1 to about 6, such as N,N-dimethylcarbamoyl, N,N-diethylcarbamoyl, N,N-dipropylcarbamoyl, N,N-dibutylcarbamoyl, N-ethyl-N-methylcarbamoyl, etc., and groups forming 5- or 6-membered ring structure (e.g. N-pyrrolidinylcarbonyl, piperidinocarbonyl by combining dialkyl moieties together. As the N-lower alkylcarbamoyl group, mention is made of N-alkylcarbamoyl groups, the carbon number of the alkyl moiety of which ranges from about 1 to about 6, such as N-methylcarbamoyl, N-ethylcarbamoyl, N-propylcarbamoyl, N-butylcarbamoyl, etc. As the halogeno group, mention is made of halogeno groups such as chloro, fluoro, bromo, iodo, etc. As the acyloxy group, mention is made of alkanoyloxy groups, the carbon number of which ranges from about 2 to about 6, such as acetoxy, propanoyloxy, butyryloxy, pivaloyloxy, etc., and benzoyloxy group. As the lower alkylsulfonylamino group, mention is made of alkylsulfonylamino groups, the carbon number of which ranges from about 1 to about 6, such as methanesulfonylamino, ethanesulfonylamino, etc. Examples of the acylamino group include alkanoylamino groups, whose carbon number ranges from about 2 to about 6, such as acetamido, propanoylamino, butyrylamino, pivaloylamino, etc. and benzamido group. As the lower alkoxycarbonylamino group, mention is made of alkoxycarbonylamino groups, the carbon number of the alkoxy moiety of which ranges from about 1 to about 6, such as methoxycarbonylamino, ethoxycarbonylamino, propoxycarbonylamino, butoxycarbonylamino, etc. As the acyl group, mention is made of alkanoyl groups, the carbon number of which ranges from about 2 to about 6, such as acetyl, propanoyl, butyryl, pivaloyl, etc., and benzoyl group. As the lower alkylthio group, mention is made of alkylthio groups, the carbon number of which ranges from about 1 to about 6, such as methylthio, ethylthio, propylthio, butylthio, etc. As the lower alkylsulfinyl group, mention is made of alkylsulfinyl groups, whose carbon number ranges from about 1 to about 6, such as methylsulfinyl, ethylsulfinyl, propylsulfinyl, butylsulfinyl, etc. As the lower alkylsulfonyl group, mention is made of alkylsulfonyl groups, the carbon number of which ranges from about 1 to about 6, such as methylsulfonyl, ethylsulfonyl, propylsulfonyl, butylsulfonyl, etc.

As the aryl group shown by $R^{11}$, mention is made of, for example, phenyl group, aromatic condensed di- or tri-cyclic hydrocarbon residue formed by 5- to 8-membered ring, such as naphthyl(1-naphthyl, 2-naphthyl), azulenyl, heptalenyl, indacenyl(as,s), acenaphthylenyl, phenanthryl, anthryl, benzocyclooctenyl, etc., hetero monocycles such as thienyl, furanyl, etc., hetero dicycles such as benzothienyl, isobenzothienyl, benzofuranyl, isobenzofuranyl, benzoxepinyl, benzothiepinyl, etc. The above-mentioned aryl groups may be partially saturated, and the partially saturated aryl groups are exemplified by indanyl(4-indanyl, 5-indanyl, etc.), indenyl(1H-inden-4-yl, 1H-inden-5-yl, etc.), dihydronaphthyl(5,6-dihydro-1-naphthyl, 5,6-dihydro-2-naphthyl, 7,8-dihydro-1-naphthyl, 7,8-dihydro-2-naphthyl, etc.), tetrahydronaphthyl(5,6,7,8-tetrahydro-1-naphthyl, 5,6,7,8-tetrahydro-2-naphthyl, etc.), 1,2,3,4-tetrahydro-1-quinolyl, 1,2,3,4-tetrahydro-2-isoquinolyl, etc.

The aryl group and the partially saturated aryl groups may have about 1 to about 4 substitutents (preferably 1 or 2), such as a lower alkyl group, a halogeno lower alkyl group, a hydroxy lower alkyl group,

an alkoxy lower alkyl group, a lower alkoxy lower alkyl group, a lower alkoxy group, a halogeno lower alkoxy group, a lower alkoxy carbonyl lower alkoxy group, a lower alkenyloxy group, an aralkyloxy group, a lower alkoxy lower alkoxy group, a lower alkoxycarbonyl, carboxyl group, carbamoyl group, an N,N-di-lower alkylcarbamoyl group, N-lower alkylcarbamoyl group, halogeno group, cyano group, nitro group, hydroxyl group, acyloxy group, amino group, a lower alkylsulfonylamino group, acylamino group, a lower alkoxycarbonylamino group, acyl group, mercapto group, a lower alkylthio group, a lower alkylsulfinyl group, a lower alkylsulfonyl group, oxo group, etc. When the aryl group and the partially saturated aryl groups have two or more substituents, the kinds of these substituents may be the same or different from one another. Practical examples of the above substituents include those shown $R^{11}$, such as cycloalkyl group, bicycloalkyl group, tricycloalkyl group, a bicyclic hydrocarbon residue, a tricyclic hydrocarbon residue or groups similar to substituents at those groups having unsaturated bond.

A divalent chain group shown by Y includes divalent functional groups shown by the following formulae:

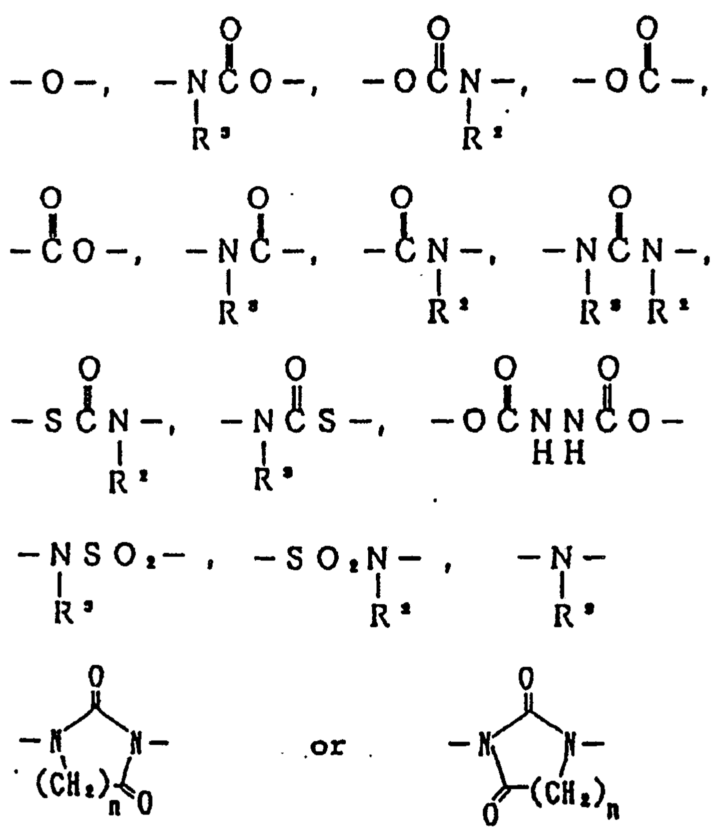

or

[wherein n denotes 1 or 2, $R^2$ and $R^3$ each stand for hydrogen, a lower alkyl group, acyl group or aryl group, and $R^3$ may be combined with $R^4$ to form a ring.]

A divalent chain group shown by Z includes divalent functional groups are shown by the following formulae:

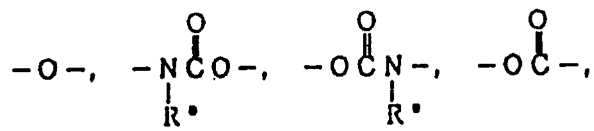

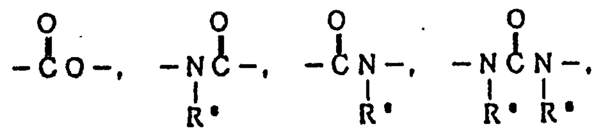

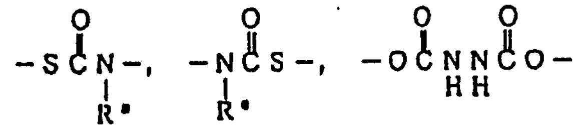

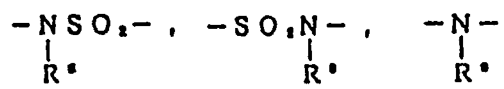

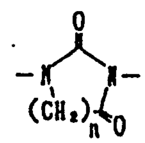

or

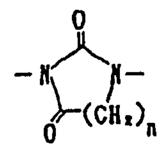

[wherein n denotes 1 or 2, $R^8$ and $R^9$ each stand for hydrogen, a lower alkyl group, acyl group or aryl group, and $R^9$ may be combined with $R^{11}$ to form a ring].

Examples of the lower alkyl groups shown by $R^1$, $R^2$, $R^3$, $R^8$ and $R^9$ include straight-chain or branced alkyl groups having about 1 to about 6 carbon atoms, such as methyl, ethyl, propyl, butyl, isobutyl, sec-butyl, tert-butyl, pentyl, hexyl, etc. The lower alkyl groups may have an unsaturated bond, and the unsaturated lower alkyl groups are exemplified by lower alkenyl groups whose carbon number ranges from about 2 to about 6, such as vinyl, allyl, 2-butenyl, 3-butenyl, etc.

Examples of the acyl group shown by $R^1$, $R^2$, $R^3$, $R^8$ or $R^9$ include lower alkanoyl groups, the carbon number of which ranges from about 2 to about 6, such as acetyl, propanoyl, butyryl, pyvaloyl, etc. and aromatic carbonul groups (e.g. benzoyl, etc.).

Examples of the aryl group shown by $R^1$, $R^2$, $R^3$, $R^8$ or $R^9$ include aromatic monocyclic, dicyclic or tricyclic hydrocarbon residues, such as phenyl, 1-naphthyl, 2-naphthyl, phenanthryl, anthryl, etc., and aromatic monocyclic or dicyclic hetero-ring, such as thienyl, furyl, benzothienyl, benzofuranyl, etc. The aryl group may have 1 to 4 substituents (preferably 1 or 2) such as halogeno group, a lower alkyl group, a lower alkoxy group, nitro group, cyano group, oxo group, hydroxyl group, amino group, a lower alkoxy carbonyl group, carbamoyl group, a lower alkylcarbamoyl group, etc. As the halogeno group, mention is made of fluoro, bromo, chloro, iodo, etc. As the lower alkyl group, mention is made of alkyl groups whose carbon number ranges from about 1 to about 6, and the lower alkyl groups may optionally have unsaturated bond. As the lower alkyl group having an unsaturated bond, mention is made of lower alkenyl groups whose carbon number ranges from about 2 about 6. Practical examples of the alkyl groups whose carbon number ranges from about 1 to about 6 and of the alkenyl groups whose carbon number ranges from about 2 to about 6 include groups similar to lower alkyl groups as the above-mentioned substituents at pyridinium ring. As the lower alkoxy group, mention is made of alkoxy groups whose carbon number ranges from about 1 to about 6. As the lower alkoxycarbonyl group, mention is made of alkoxycarbonyl groups, the carbon number of the alkoxy moiety of which ranges from about 1 to about 6. As the lower alkylcarbamoyl group, mention is made of N-alkylcarbamoyl groups, the carbon number of the alkyl moiety of which ranges from about 1 to about 6, and of N,N-dialkylcarbamoyl groups, the carbon number of each alkyl moiety of which ranges from about 1 to about 6. Practical examples of these groups include groups similar to a lower alkoxy group, a lower alkoxycarbonyl group and a lower alkylcarbamoyl group as the above-mentioned substituents at pyridinium ring. When U is a group of the formula

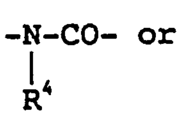 or 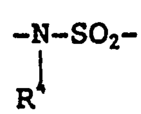 ,

$R^2$ and $R^4$ may be combined to form a ring. Specifically, as

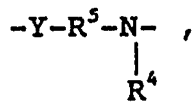

mention is made of groups shown by the following formulae:

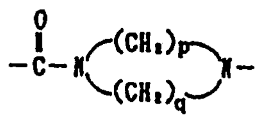,
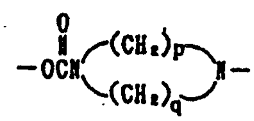,

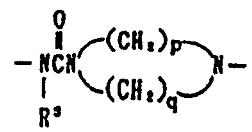,
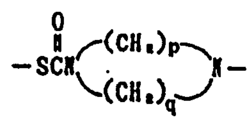,

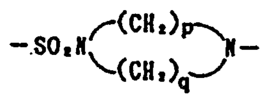
or
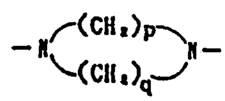

[wherein p and q respectively stand for 2 or 3.]

$R^9$ and $R^{11}$ may be combined to form a ring. As the ring represented by the formula

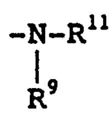

mention is made of, for example, 3- to 8-membered monocyclic hetero-ring, such as 1-aziridinyl, 1-azetidinyl, piperidino, perhydro-1-azepinyl, perhydro-1-azocinyl, morpholino, thiomorpholino, 1-piperazinyl, 3-thiazolidinyl, etc., condensed bicyclic or cross-linked bicyclic hetero-ring, such as 1-indolyl, perhydro-1-indolyl, 2-isoindolyl, perhydro-2-isoindolyl, 1,2,3,4-tetrahydro-1-quinolyl, 1,2,3,4-tetrahydro-2-isoquinolyl, pehydro-1-quinolyl, perhydro-2-isoquinolyl, 3-azabicyclo[3,2,2]non-3-yl, etc., and condensed tricyclic hetero-ring such as 9-carbazolyl, 10-acryldanyl

( 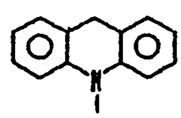 ),

10,11-dihydro-5H-5-dibenz[b,f]azepinyl, 5,6,11,12-tetrahydro-5-dibenz[b,f]azocinyl, 1,2,3,4-tetrahydro-9-carbazolyl, 10-phenoxazinyl, 10-phenothiazinyl, etc.

The above-mentioned hetero ring may have about one to about four (preferably one or two) substituents such as, among others, a lower alkyl group, a halogeno lower alkyl group, a hydroxy lower alkyl group, an acyloxy lowr alkyl group, a lower alkoxy lower alkyl group, a lower alkoxy group, a halogeno lower alkoxy group, a lower alkoxy carbonyl-lower alkoxy group, a lower alkenyloxy group, aralkyloxy group, a lower alkoxy-lower alkoxy group, a lower alkoxycarbonyl group, carboxyl group, carbamoyl group, an N,N-di-lower alkylcarbamoyl group, an N-lower alkyl carbamoyl group, halogeno group, cyano group, nitro group, hydroxyl group, acyloxy group, amino group, a lower alkylsulfonylamino group, acylamino group, a lower alkoxycarbonylamino group, acyl group, mercapto group, a lower alkylthio group, a lower alkylsulfinyl group,

a lower alkylsulfonyl group and oxo group. When the hetero ring has two or more substituents, the kinds of these substituents may be the same as or different from one another.

Lower alkyl groups as the above-mentioned substituents are exemplified by alkyl groups whose carbon number ranges from 1 to about 6, such as methyl, ethyl, propyl, isopropyl, butyl, isobutyl, sec-butyl, tert-butyl, etc. As the halogeno lower alkyl group, mention is made of alkyl groups whose carbon number ranges from about 1 to about 6, which are substituted with 1 to 3 halogeno groups such as trifluoromethyl, fluoromethyl, chloromethyl, chloroethyl, fluoroethyl, etc. As the hydroxy lower alkyl group, mention is made of hydroxy alkyl groups whose carbon number ranges from about 1 to about 6, such as hydroxymethyl, hydroxyethyl, hydroxypropyl, hydroxybutyl, etc. As the acyloxy lower alkyl group, mention is made of alkyl groups whose carbon number ranges from about 1 to about 6, which are substituted with, for example, a lower alkanoyloxy group whose carbon number ranges from about 2 to about 6, or a benzoyloxy group such as acetoxyethyl, benzoyloxyethyl, etc. As the lower alkoxy-lower alkyl group, mention is made of alkyl groups whose carbon number ranges from about 1 to about 6, which are substituted with, for example, an alkoxy group whose carbon number ranges from about 1 to about 6 such as methoxyethyl, ethoxyethyl, propoxyethyl, butoxyethyl, methoxypropyl, methoxybutyl, ethoxypropyl, ethoxybutyl, etc. As the lower alkoxy group, mention is made of alkoxy groups whose carbon number ranges from about 1 to about 6 such as methoxy, ethoxy, propoxy, isopropoxy, butoxy, isobutoxy, sec-butoxy, tert-butoxy, etc. As the halogeno lower alkoxy group, mention is made of alkoxy groups whose carbon number ranges from about 1 to about 6, which are substituted with 1 to 3 halogeno groups such as chloroethoxy, fluoroethoxy, difluoroethoxy, trifluoroethoxy, chloropropoxy, chlorobutoxy, etc. As the lower alkoxy carbonyl-lower alkoxy group, mention is made of alkoxy groups whose carbon number ranges from about 1 to about 6, which are substituted with an alkoxycarbonyl group, the carbon number of the alkoxy moiety of which ranges from about 1 to about 6, such as methoxycarbonylmethoxy, ethoxycarbonylmethoxy, butoxycarbonylmethoxy, methoxycarbonylpropoxy, ethoxycarbonylethoxy, etc. Examples of the lower alkenyloxy group include alkenyloxy groups whose carbon number ranges from about 2 to about 6, such as vinyloxy, allyloxy, butenyloxy, etc. As the aralkyloxy group, mention is made of phenyl lower alkyloxy groups, the carbon number of the lower alkyl moiety of which ranges from about 1 to about 6, such as benzyloxy, phenethyloxy, 3-phenylpropyloxy, α-methylphenethyloxy, α-methylbenzyloxy, α-ethylbenzyloxy, β-ethylphenethyloxy, β-methylphenethyloxy, etc. As the lower alkoxy-lower alkoxy group, mention is made of alkoxy groups whose carbon number ranges from about 1 to about 6, which are substituted with, for example, an alkoxy group whose carbon number ranges from 1 to about 6, such as ethoxymethoxy, methoxyethoxy, butoxyethoxy, ethoxypropoxy. Examples of the lower alkoxycarbonyl group include alkoxycarbonyl groups, the carbon number of the alkoxy moiety of which ranges from 1 to about 6, such as methoxycarbonyl, ethoxycarbonyl, propoxycarbonyl, butoxycarbonyl, etc. As the N,N-di-lower alkylcarbamoyl group, mention is made of N,N-dialkylcarbamoyl groups, the carbon number of each alkyl moiety of which ranges from about 1 to about 6, such as N,N-dimethylcarbamoyl, N,N-diethylcarbamoyl, N,N-dipropylcarbamoyl, N,N-dibutylcarbamoyl, N-ethyl-N-methylcarbamoyl, etc., and groups forming 5- or 6-membered ring structure (e.g. pyrrolidinylcarbonyl, piperidinocarbonyl) by combining dialkyl moieties together. As the N-lower alkylcarbamoyl group, mention is made of N-alkylcarbamoyl groups, the carbon number of the alkyl moiety of which ranges from about 1 to about 6, such as N-methylcarbamoyl, N-ethylcarbamoyl, N-propylcarbamoyl, N-butylcarbamoyl, etc. As the halogeno group, mention is made of halogeno groups such as chloro, fluoro, bromo, iodo, etc. As the acyloxy group, mention is made of alkanoyloxy groups, the carbon number of which ranges from about 2 to about 6, such as acetoxy, propanoyloxy, butyryloxy, pivaloyloxy, etc., and benzoyloxy. As the lower alkylsulfonylamino group, mention is made of alkylsulfonylamino groups, the carbon number of which ranges from 1 to about 6, such as methanesulfonylamino, ethanesulfonylamino, etc. Examples of the acylamino group include alkanoylamino groups, whose carbon number ranges from about 2 to about 6, such as acetamido, propanoylamino, butylylamino, pivaloylamino, etc., and benzamido group. As the alkoxycarbonylamino group, the carbon number of the alkoxy moiety of which ranges from about 1 to about 6, such as methoxycarbonylamino, ethoxycarbonylamino, propoxycarbonylamino, butoxycarbonylamino, etc. As the acyl group, mention is made of alkanoyl groups, the carbon number of which ranges from about 2 to about 6, such as acetyl, propanoyl, butyryl, pivaloyl, etc., and benzoyl group. As the lower alkylthio group, mention is made of alkylthio groups, the carbon number of which ranges from about 1 to about 6, such as methylthio, ethylthio, propylthio, butylthio, etc. As the lower alkylsulfinyl group, mention is made of alkylsulfinyl groups, the carbon number of which ranges from about 1 to about 6, such as-methylsulfinyl, ethylsulfinyl, propylsulfinyl, butylsulfinyl, etc. As the lower alkylsulfonyl group, mention is made of alkylsulfonyl groups, the carbon number of which ranges from about 1 to about 6, such as methylsulfonyl, ethylsulfonyl, propylsulfonyl, butylsulfonyl, etc.

As the pyridinium ring

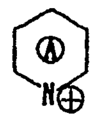

formed by condensation of aromatic ring, mention is made of, for example, quinolinium group and isoquinolinium group.

Examples of the counter anion shown by $W^{\theta}$ include pharmacologically acceptable anions, for example, anions of an inorganic acid such as chloride ion, bromide ion, iodide ion, sulfate ion, nitrate ion, phosphate ion, etc. and anions of an organic acid such as acetate ion, tosylate ion, mesylate ion, etc.

Among the above-mentioned compounds, preferable ones are those representable by the following formula:

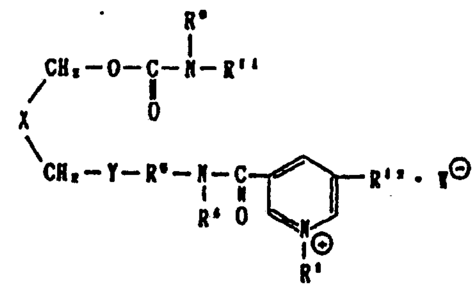

[wherein $R^1$ stands for a lower alkyl, $R^4$ stands for a phenyl group optionally substituted with a halogeno group, $R^5$ stands for ethylene group or trimethylene group, $R^{11}$ stands for an alkyl group whose carbon number is 1 to 30, a cyclo-alkyl group whose carbon number is 3 to 8, phenyl group or naphthyl group, $R^{12}$ stands for a halogeno group, X stands for $-(CH_2)_m-$ (wherein m denotes 0 or 1), $W^{\theta}$ stands for a halogeno ion, Y stands for a group of the formula
$-NH-\underset{O}{\overset{\|}{C}}-$ or $-NH-\underset{O}{\overset{\|}{C}}-O-$,
and $R^9$ stands for hydrogen or

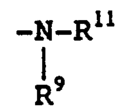

stands for piperidino, morpholino or 1,2,3,4-tetrahydro-2-isoquinolyl when $R^9$ is bonded to $R^{11}$].

Pyridinium derivatives [VII] can be synthesized, for example, in accordance with the methods disclosed in the specification of Japanese Patent Application No. 186494/1988 or European Patent Publication EP-A-0301751.

As representative compounds, there are mentioned the following compounds.

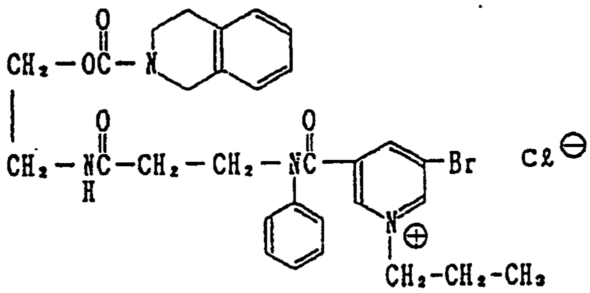

[VIIIa]

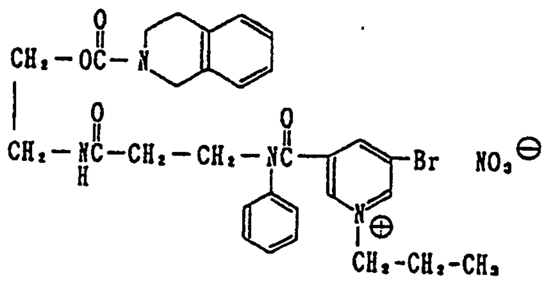

[VIIIb]

5) Benzodiazepine derivatives

There are mentioned etizolam which is disclosed in Chem. Pharm. Bull., 35, 2119 (1987), alprazolam and triazolam which are disclosed in Science, 226, 1454 (1984), and brotizolam which is disclosed in New Horizons in Platelet Activating Factor Research, 38 (October 15-18, 1985) [South Carolina, United States of America], and, as a representative compound, there is mentioned WEB2086 which is disclosed in Immunopharmacology, 13, 117 (1987).

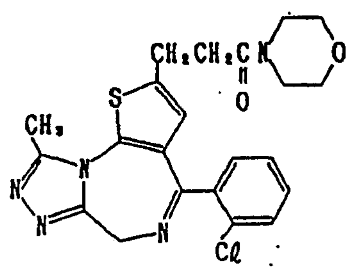

[IX]

(WEB2086)

In addition, the following compounds can also be used as PAF antagonists.

Compounds disclosed in Japanese Unexamined Patent Publication No. 104066/1985, for instance, a compound of the formula:

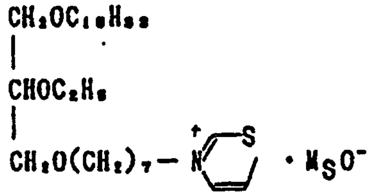

[X]

[wherein MSO⁻ is mesylate.]

Compounds disclosed in Japanese Unexamined Patent Publication No. 158172/1985, for instance, a

compound of the formula:

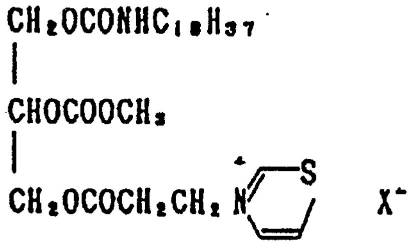

[XI]

[wherein $X^-$ is an anion (e.g. chloride, bromide).]
SRI63-072 which is disclosed in
Immunopharmacology, 12, 11 (1986)

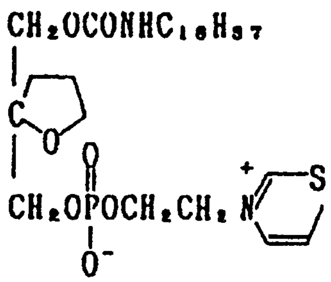

[XII]

(SRI63-072)

SRI63-441 which is disclosed in Thromb. Haemost., 56, 40 (1986)

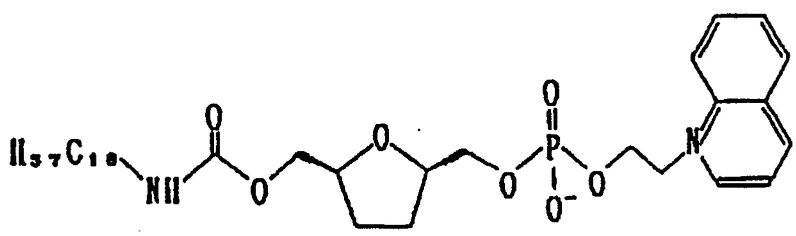

[XIII]

(SR163-441)

Compounds disclosed in Japanese Unexamined Patent Publication No. 224682/1985, for instance, Kadsurenone of the formula:

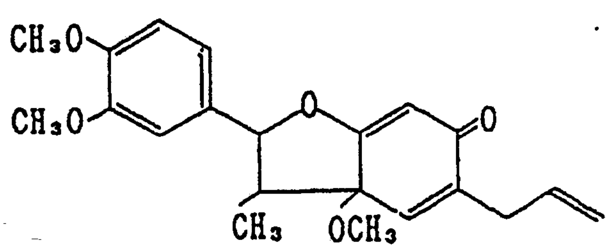

[XIV]

Compounds disclosed in Japanese Unexamined Patent Publication No. 116679/1985, for instance, a compound of the formula:

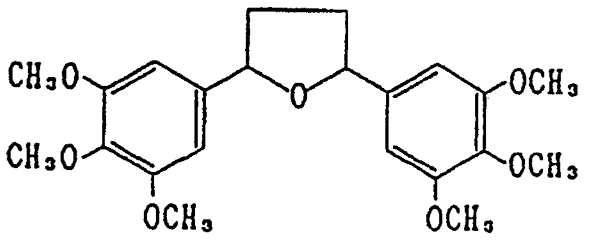

[XV]

Compounds disclosed in Immunopharmacology, 10, 69 (1985), for instance, Ginkgolide B [BN-52021].
Compounds disclosed in Japanese Unexamined Patent Publication No. 14932/1985.
Compounds disclosed in Japanese Unexamined Patent Publication No. 208975/1985.
A compound of the formula:

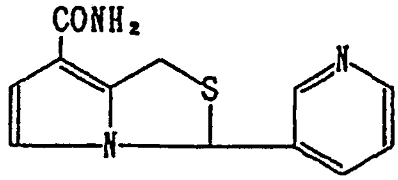

[XVI]

which is disclosed in "Is there a case for PAF-acether antagonists?" Abstract A2 (1985).
FR-900452 which is disclosed in Thromb. Res., 42, 661 (1986).

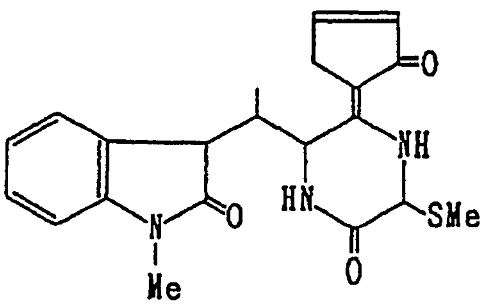

[XVII]

(FR-900452)

When the PAF antagonists are administered as agents for prevention or treatment of hyperendothelinemia, the dosage varies depending upon the kind of PAF anatagonists, the subject to be administered, symptoms and so on. For example, the compounds [I], [IIIa], [IIIb] and [V] are mentioned below.

The compounds [I], [V] and their salts are so hydrophilic and lipophilic and so low in toxicity, that they can therefore be safely administered orally or non-orally, as they are in a form of powder or as a pharmaceutical composition, when they are used as agents for prevention or treatment of hyperendothelinemia. The dosage varies upon the subject to be administered, symptoms and route of administration, and when the compound [I] is administered through intravenous injection to a human adult, for example, it can be advantageously administered usually in a single dose in the range of about 0.1 to 50 mg/kg body weight, preferably in the range of about 1 to 40 mg/kg body weight, more preferably in the range of 1 to 10 mg/kg body weight, about once to 3 times a day. When the compound [V] is administered to a human adult for prevention or treatment of hyperendothelinemia, it can be advantageously administered usually in a single dose in the range of about 0.01 to 20 mg/kg body weight, preferably in the range of about 0.1 to 10 mg/kg body weight, more preferably in the range of 0.1 to 2 mg/kg body weight, about once to 3 times a day. The dosages for other non-oral routes as well as the oral dosage may be selected referring to the above dose-level. When the symptoms are very serious, dosage increases may be made according to the severity of the symptoms.

The compounds [IIIa], [IIIb] and their salts are also so low in toxicity, that they can be administered orally or non-orally to a mammal (e.g. human, rabbit, dog, cat, rat, mouse) as they are in a form of powder

or as a pharmaceutical composition. The dosage varies upon the subject to be administered, disease to be treated, symptoms and route of administration, and for the purpose of prevention or treatment of hyperendothelinemia in a human adult, the compound [IIIa], [IIIb] or a salt thereof can be advantageously administered through intravenous injection usually in a single dose in the range of 0.01 to 20 mg/kg body weight, preferably in the range of 0.1 to 10 mg/kg body weight, more preferably in the range of 0.1 to 2 mg/kg body weight, about once to 5 times a day, preferably about once to 3 times a day. In addition, the compound [IIIa], [IIIb] or a salt thereof can be administered through drip infusion in a single dose in the range of about 0.01 to 1.0 mg/kg body weight/min. over a period of about 1 hour, about once to 5 times a day, preferably about once to 3 times a day. The dosages for other non-oral routes as well as the oral dosage may be selected referring to the above dose-levels. When symptoms are very serious, dosage increases may be made according to the severity of the symptoms.

When the compound [IIIa], [IIIb] or a salt thereof is administered orally for prevention or treatment of hyperendothelinemia in a human adult, for example, it can be advantageously administered usually in a single dose in the range of about 0.05 to 20 mg/kg body weight, preferably in the range of 0.2 to 5 mg/kg body weight, about once to 5 times a day, preferably about once to 3 times. The dosages for other non-oral routes may be selected referring to the above dose-levels.

The PAF antagonists are administered as they are or as a suitable pharmaceutical composition. The pharmaceutical composition to be used for the above administration comprises an effective amount of the compound [I], [IIIa], [IIIb], [V] or a salt thereof as the active ingredient, and a pharmaceutically acceptable carrier, diluent or excipient therefor, and the composition is provided in a dosage form suitable for oral or non-oral administration.

The composition for oral administration includes, for example, solid or liquid dosage forms, and as their specific examples, there may be mentioned tablets (inclusive of sugar-coated tablets and film-coated tablets), pills, granules, powders, capsules (inclusive of soft capsules), syrups, emulsions, suspensions, etc. Such compositions can be manufactured by the *per se* known procedures and comprise carriers and excipients commonly used in the pharmaceutical industry. Examples of the carriers and excipients for the preparation of tablets include lactose, starch, sugar and magnesium stearate, etc.

The compositions for non-oral administration include for example, injections and suppositories, and as examples of the injection, there may be mentioned dosage forms, such as injectable solutions for intravenous injection, for subcutaneous injection, for intracutaneous injection, for intramuscular injection and for drip injection. Such injectable solutions are prepared by a *per se* known procedure, for example, by dissolving, suspending or emulsifying the compound [I], [IIIa], [IIIb], [V] or a salt thereof in a sterile aqueous or oily solution usually used for injectable solutions. The aqueous solution for injection includes, for example, physiological saline solution, isotonic solution containing glucose and other adjuvants, and may be employed in combination with a suitable solubilizer, such as alcohols (e.g., ethanol, etc.), polyalcohols (e.g., propylene glycol, polyethylene glycol, etc.), and nonionic surface active agents [e.g., polysorbate 80, HCO-50 (polyoxyethylene (50 mol) adduct of hydrogenated castor oil), etc.]. The oily solution for injection includes, for example, sesame oil and soybean oil, and may be used in combination with such a solubilizer as benzyl benzoate and benzyl alcohol. The injectable solution prepared is usually filled into suitable ampoules to be supplied as an injection. The suppositories for rectal administration are produced by a *per se* known procedure, for example, by incorporating the compound [I], [IIIa], [IIIb], [V] or a salt thereof into a usual suppository base, followed by compression into a shape.

The above oral or non-oral pharmaceutical compositions are conveniently processed into unit dosage forms commensurate with the dose of the active ingredient. Such dosage forms include tablets, pills, capsules, injections (ampules), suppositories, etc. and desirably contain generally 5 to 500 mg of the compound [I], [IIIa], [IIIb] or [V] per dose unit, preferably 5 to 100 mg in the case of injections and 10 to 250 mg in the case of other dosage forms.

Unless an undesirable interaction is induced, the above compositions may contain another active ingredients in addition to the compound [I], [IIIa], [IIIb] or [V].

Other PAF antagonists can be also administered by a method of administration and a dose similar to those of the compounds [I], [IIIa], [IIIb] and [V].

Endothelin which has a potent activity on contraction of vascular and bronchi smooth muscle induces hypertension and bronchoconstriction, and in a higher concentration (about 0.1 to 5 nmol per 100 ml of blood) induces various diseases such as ischemic cerebropathies and cardiopathies (e.g. cerebral apoplexy, angina pectoris, myocardial infarction, heart failure, arrhythmia), renal disorders (e.g. renal inflammation), circulatory disorders of various organs (e.g. liver, lung, intestine) and asthma and causes death in animal individuals in some cases.

PAF antagonists can be administered as effective agents for prevention or treatment of the above

diseases (hyperendothelinemia) induced by excess secretion of endothelin.

More specifically, the following experiments show that PAF antagonists are effective agents for prevention or treatment of hyperendothelinemia.

Experiment 1

Protective effects of PAF anatagonists against endothelin-induced death

[Method]

Male SD-Jcl rats (5-6 weeks old) and male Jcl-ICR mice (5-6 weeks 01d) were used without anesthetization.

Physiological saline or drug-dissolved physiological saline was given via the tail vein to a rat in a volume of 1 ml/kg and to a mouse in a volume of 5 ml/kg. Five minutes later, the same volume of endothelin (Peptide Institute; 5 nmol/kg)-dissolved physiological saline was intravenously given.

In the case of oral administration of a drug, the drug was given in 10 ml/kg of 5% gum arabic suspension one hour before the administration of endothelin.

One hour after the administration of endothelin, the survival rates were measured, and the effect of the drug was evaluated.

[Results]

The results are shown in Table 1.

TABLE 1

Protective effects of PAF antagonists against endothelin-induced death in rats and mice

| Sp. | Group | Dose (mg/kg) | Route | Protective effect to death: Survival/Total | Survival Rate (%) |
|---|---|---|---|---|---|
| rat | | | | | |
| | Control | - | - | 8/56 | 14 |
| | Compd [II] | 3 | i.v. | 15/16 | 94** |
| | Compd [IX] | 30 | p.o. | 6/15 | 40** |
| mouse | | | | | |
| | Control | - | - | 10/90 | 11 |
| | Compd [II] | 3 | i.v. | 10/10 | 100** |
| | Compd [VI] | 30 | i.v. | 5/14 | 36* |
| | Compd [IV] | 100 | p.o. | 15/15 | 100** |
| | Compd [IX] | 30 | p.o. | 8/15 | 53** |

Statistical analysis : $X^2$-test as compared with the control group

* $P<0.05$

**$P<0.01$

Experiment 2

Protective effects of PAF antagonists against arrhythmia-inducing activity of endothelin

[Method]

Male SD-Jcl rats (9 weeks old) were anesthetized with pentobarbital (50 mg/kg, intraperitoneal injection), and electrocardiogram (Lead II) was monitored on a polygraph. A drug was dissolved in physiological saline and given via the tail vein in a volume of 1 ml/kg. Five minutes later, endothelin (5 nmol/kg) was intravenously administered. In the case of oral administration, a drug was given in 10 ml/kg of 5% gum arabic suspension one hour before the administration of endothelin.

[Results]

Table 2 shows effects of PAF antagonists (compound [II], compound [IX]) on premature ventricular beats (PVB), and incidences of ventricular tachycardia (VT) and ventricular fibrillation (VF) on the electrocardiogram, which are observed after the administration of endothelin. Endothelin caused abnormal electrocardiogram changes in most of the rats, and the compounds [II] and [IX] inhibited the above incidences.

TABLE 2

| Protective effects of PAF antagonists on arrhythmia-inducing activity of endothelin in rats | | | | | | |
|---|---|---|---|---|---|---|
| Group | Number of rats | Dose (mg/kg) | Route | PVB counts (15 min.) | VT incidence | VF incidence |
| Control | 10 | - | - | 19±3 | 10/10 | 10/10 |
| Compd [II] | 9 | 1 | i.v. | 9±8** | 3/9** | 2/9** |
| Compd [IX] | 6 | 30 | p.o. | 12±5* | 4/6** | 4/6** |
| Statistical analysis : Wilcoxon rank sum test (PVB) and $X^2$-test (VT,VF) were used. | | | | | | |

* P<0.05, **P<0.01, ***P<0.001 as compared with the control group

Experiment 3

Inhibiting activities of PAF antagonists against elevation of cytosolic calcium concentration induced by endothelin

[Method]

A glass cover slide coated with 4 $\mu$M fura-2-loaded cultured rat vascular smooth muscle cells was inserted into a quartz cuvette which contained 2.5 ml HEPES buffer (pH 7.2). On this occasion, the glass cover slide was fixed on a diagonal of 45° to the direction of excitation light.

The fluoroscence was measured in a Hitachi F-4000 spectrofluorimeter. Wavelengths were set at 340 nm and 380 nm for excitation and 505 nm for emission.

Fmax and Fmin were estimated after addition of ionomycin (2 $\mu$M; Calbiochem) as calcium ionophore and EGTA (8 mM) as calcium ion-chelator, respectively.

The cytosolic calcium ion concentration $[Ca^{++}]_i$ values were calculated by Tsien et al. formula: $[Ca^{++}]_i$ = kd•(F-Fmin)/(Fmax-F) with modifications assuming kd = 224 nM, i.e. by the formulas:

$[Ca^{++}]_i$ = kd•(R-Rmin)•$Sf_2$/(Rmax-R)•$Sb_2$

R = [D($W_1$)-Auto$F_1$]/[D($W_2$)-Auto$F_2$]

D($W_1$) : Determined value at wavelength for excitation $W_1$

D($W_2$) : Determined value at wavelength for excitation $W_2$

Auto$F_1$ : self-fluoroscence at $W_1$

Auto$F_2$ : self-fluoroscence at $W_2$

Rmin : R in zero $Ca^{++}$ = Fmin ($W_1$)/Fmin ($W_2$)

Rmax : R in $Ca^{++}$-saturated concentration = Fmax ($W_1$)/Fmax ($W_2$)

$Sf_2$ : strength of fluoroscence by free $Ca^{++}$ at $W_2$

$Sb_2$ : strength of fluoroscence by bound $Ca^{++}$ at $W_2$

The experiments were carried out as follows:

PAF antagonists, the compounds [II], [IV] and [VIIIa], were added to the solution in the cuvette. Incubation was conducted for 5 minutes and then endothelin (4 x $10^{-9}$ M) was added to observe changes of strength of fluoroscence.

[Results]

Endothelin induced a two-phase increase of cytosolic calcium concentration, i.e. a transient elevation and subsequently a sustained elevation.

It was found that the compounds [II], [IV] and [VIIIa] as PAF antagonists could inhibit both of the transient elevation and the sustained elevation of cytosolic calcium concentration by endothelin.

Tables 3 and 4 show the inhibitory activities at various concentrations of the compounds.

TABLE 3

| Inhibitory activities of PAF antagonists against endothelin-induced transient increase of cytosolic calcium concentration | | |
|---|---|---|
| Compound | Concentration (M) | Inhibitory activity (%) |
| [II] | $4 \times 10^{-7}$ | 100 |
| | $4 \times 10^{-6}$ | 100 |
| [IV] | $4 \times 10^{-7}$ | 76.2 |
| [VIIIa] | $4 \times 10^{-7}$ | 64.2 |
| | $4 \times 10^{-6}$ | 78.2 |

TABLE 4

Inhibitory activities of PAF antagonists against endothelin-induced sustained increase of cytosolic calcium concentration

| Compound | Concentration (M) | Inhibitory activity (%) |
|---|---|---|
| [II] | $4 \times 10^{-8}$ | 100 |
| | $4 \times 10^{-7}$ | 100 |
| [IV] | $4 \times 10^{-7}$ | 52.6 |
| [VIIIa] | $4 \times 10^{-8}$ | 72.1 |
| | $4 \times 10^{-7}$ | 100 |

Experiment 4

Acute toxicities

[Method]

PAF antagonists were administered to male Wistar rats (4-6 weeks old) to observe the acute toxicities.

[Results]

Table 5 shows the $LD_{50}$ values (mg/kg) of each of the PAF antagonists.

TABLE 5

| Acute toxicities of PAF antagonists | | |
|---|---|---|
| PAF antagonists | Route | $LD_{50}$ (mg/kg) |
| Compd [II] | i.v. | > 20 |
| Compd [IV] | p.o. | >1000 |
| Compd [VI] | i.v. | > 50 |

Experiment 5

Effect on acute renal failure in rats

[Method]

Male Spraque Dawley (Jcl) rats, 5 weeks old, were anesthetized with sodium pentobarbital (50 mg/kg, i.p.) and the renal artery was occluded bilaterally for 45 minutes followed by reperfusion for 20 hours. The blood was collected from the abdominal aorta under anesthesia for measurement of blood urea nitrogen (BUN).

[Results]

The concentration of BUN was markedly elevated 20 hours after reperfusion. The compound [VIIIb] at an oral dose of 30 mg/kg, which was given 1 hour prior to the occlusion of renal artery, inhibited significantly the elevation of BUN (Table 6).

TABLE 6

| Effect of PAF antagonist on acute renal failure in rats | | |
|---|---|---|
| | No of animal | Blood urea nitrogen (BUN) (mg/dl) |
| Control | 10 | 110 ± 4 |
| Compd [VIIIb] | 3 | 88 ± 2* |
| Student's t-test, | | |

* P<0.05

Experiment 6

Antiproteinuric action in glomerulonephritic rats

[Method]

Glomerulonephritis in rats was prepared according to the method by Nagamatsu et al. (Folia Pharmacol. Jpn. 78, 491, 1981). The mixture of 3 mg rabbit serum albumin (RSA) and the same amount of complete Freund's adjuvant was prepared. One ml/rat (3 mg/rat) of the mixture was first injected subcutaneously to 5 weeks old Spraque Dawley (Jcl) rats. 2 weeks later, RSA (1 mg/rat) was injected intravenously 3 times a week for 8 weeks. At the point of RSA injection for the first 4 weeks, urine was collected from the rat for 24 hours to measure urinary excretion of albumin and total protein. According to the value of albumin and total protein obtained, the rats were separated into 2 groups of the control and the treatment group. Then, the drug or vehicle was orally given once a day for 4 weeks and the 24 hour-urine at the 4 week of the dosage was collected for measurement of urinary albumin and total protein.

[Results]

The urinary excretion of albumin and total protein was markedly increased by repeated administration of RSA. The compound [IV] at the daily administration for 4 weeks inhibited the urinary excretion of albumin and total protein (Table 7).

TABLE 7

| Inhibitory action of PAF antagonist on proteinuria in glomerulonephritic rats | | | |
|---|---|---|---|
| | Dose | albumin total protein | |
| | (mg/kg, p.o.) | (mg/100 g/24 hr) | |
| Control<br>Compd [IV] | -<br>100 | 61 ± 22<br>17 ± 11 | 94 ± 31<br>43 ± 16* |
| Student's t-test, | | | |
| * P<0.05 | | | |

Experiment 7

Protective action on ischemic cerebral damage in M-SHRsp

[Method]

Male malignant spontaneously hypertensive rats (M-SHRsp), 8 weeks old, were used. The carotid artery was occluded bilaterally for 1 hour under conscious condition followed by reperfusion for 24 hours. The drug was orally given 1 hour prior to the occlusion of the artery. The survival rate of rats was estimated

during the 24 hour reperfusion.

[Results]

Eight out of 9 rats in the control group died within 24 hours of reperfusion. The compound [IV] at the oral doses of 1 to 100 mg/kg protected rats from death in a dose related manner; 6 out of 7 rats at 10 mg/kg and all out of 4 rats at 100 mg/kg survived (Table 8).

TABLE 8

| Protective action on ischemic cerebral damage in M-SHRsp | | |
|---|---|---|
| | Dose | Survival rate (24 hr) |
| | (mg/kg, p.o.) | (Rats died/Total rats) |
| Control | - | 1/9 |
| Compd [IV] | 1<br>10<br>100 | 2/7<br>6/7*<br>4/4* |
| $X^2$-test, | | |
| * $P<0.05$ | | |

The present invention will be illustrated in more detail by the following preparation examples, but the present invention should not be limited to those.

Preparation Example 1

The compound [II], i.e. 2-[N-acetyl-N-(2-methoxy-3-octadecylcarbamoyloxypropoxycarbonyl)-aminomethyl]-1-ethylpyridinium chloride (10 g) is dissolved in distilled water (1.0ℓ ). After filtration for sterilization, the solution is distributed into 1000 vials by 1 ml portions and lyophilized, followed by tightly closing.

On the other hand, distilled water (2 ℓ ) for injection containing mannitol (100 g) is distributed into 1000 ampoules for injection by 2 ml portions, followed by sealing to prepare 1000 injectable solutions.

On the occasion of use, the powder in the former vial is dissolved in the mannitol solution for injection.

Preparation Example 2

The compound [IV], i.e. 1-(2,3-dimethoxy-6,7- dihydro-5H-benzocycloheptenyl-8-carbonyl)-4-(3,4,5-trimethoxybenzoyl)piperazine (10 g), lactose (90 g) and corn starch (17 g) are mixed, and granulated with a paste prepared by corn starch (7 g). The granules are incorporated with corn starch (5 g) and magnesium stearate (1 g) and compressed to prepare 1000 tablets.

Preparation Example 3

The compound [VI], i.e. 3-(N-n-octadenylcarbamoyloxy)-2-methoxypropyl 2-thiazolioethyl phosphate (10 g) is dissolved in distilled water (1.0ℓ ). After sterile filtration, the solution is distributed into 1000 vials by 1 ml portions and lyophilized, followed by tightly closing.

On the other hand, distilled water (2ℓ ) for injection containing xylitol or mannitol (100 g) is distributed into 1000 ampoules for injection by 2 ml portions, followed by sealing to prepare 1000 injectable solutions.

On the occasion of use, the powder in the former vial is dissolved in the xylitol (or mannitol) solution for injection.

## Claims

1. Use of a platelet activating factor antagonist for manufacturing a drug for prevention or treatment of hyperendothelinemia.

2. Use according to claim 1, wherein the hyperendothelinemia is ischemic cerebropathy, ischemic cardiopathy, renal disorder, circulatory disorder, asthma or sudden death.

3. Use according to claim 1, wherein the platelet activating factor antagonist is (i) a pharmaceutically acceptable salt of 2-[N-acetyl-N-(2-methoxy-3-octadecylcarbamoyloxypropoxycarbamoyl)aminomethyl]-1-ethylpyridinium with an anion, (ii) 1-(2,3-dimethoxy-6,7-dihydro-5H-benzocycloheptenyl-8-carbonyl)-4-(3,4,5-trimethoxybenzoyl]piperazine, (iii) 3-(N-n-octadecylcarbamoyloxy)-2-methoxypropyl 2-thiazolioethyl phosphate, (iv) a pharmaceutically acceptable salt of 5-bromo-3-[N-[2-[[2-(1,2,3,4-tetrahydroisoquinolyl)-carbonyloxy]ethyl]carbamoyl]ethyl-N-phenyl]carbamoyl-1-propylpyridinium with an anion, or (v) 3-[4-(2-chlorophenyl)-9-methyl-6H-thieno [3,2-f] [1,2,4]- triazolo-[4,3-a] [1,4]-diazepine-2-yl]-1-(4-morpholinyl)-1-propanone.